(19) Europäisches Patentamt
European Patent Office
Office européen des brevets

(11) **EP 3 958 271 A1**

(12) **EUROPEAN PATENT APPLICATION**

(43) Date of publication:
**23.02.2022 Bulletin 2022/08**

(21) Application number: **20191806.7**

(22) Date of filing: **19.08.2020**

(51) International Patent Classification (IPC):
**G16C 20/30** (2019.01)     **G16C 20/70** (2019.01)
**G16C 60/00** (2019.01)     **G01N 33/28** (2006.01)

(52) Cooperative Patent Classification (CPC):
**G01N 33/2823; G06N 3/00; G16C 60/00;**
G16C 20/30; G16C 20/70

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Abu Dhabi Company for Onshore Petroleum
Operation Limited
Abu Dhabi (AE)**
• **Abu Dhabi National Oil Company
Abu Dhabi (AE)**

(72) Inventors:
• **Mohan, Richard
Abu Dhabi (AE)**
• **Ghorayeb, Kassem
Abu Dhabi (AE)**
• **Mawlod, Arwa Ahmed
Abu Dhabi (AE)**
• **Mustapha, Hussein
Abu Dhabi (AE)**

(74) Representative: **Mader, Joachim
Bardehle Pagenberg Partnerschaft mbB
Patentanwälte, Rechtsanwälte
Prinzregentenplatz 7
81675 München (DE)**

(54) **HYDROCARBON FLUID PROPERTIES PREDICTION USING MACHINELEARNING-BASED MODELS**

(57) A computer-implemented method (100) for predicting hydrocarbon fluid properties using machine-learning-based models is provided. The method comprises the step of receiving (101) a minimal set of pressure-volume-temperature (PVT) data for hydrocarbon fluid samples from a PVT data base; reading (102) the minimal set of PVT data by a reader module; transforming (103) the minimal set of PVT data into a unified data structure by the reader module; selecting (104) items of the PVT data from the minimal set of PVT data by the reader module; processing (105) the selected items of the PVT data by a correlating module to identify a plurality of correlations in the selected items of the PVT data based on one or more of the fluid properties of the hydrocarbon fluid samples; clustering (106), using of at least one of a plurality of clustering algorithms, the selected items of the PVT data into a plurality of clusters by a clustering module; and performing (107) machine learning by a machine learning module on ones of the plurality of clusters to predict missing fluid properties in the minimal set of PVT data and thus to obtain a complete set of PVT data. Further a computer-implemented method (200) for generating equations of state (EoS) for a plurality of hydrocarbon fluids is provided. The method comprises the step of delumping (201) pressure-volume-temperature (PVT) data for hydrocarbon fluid samples from a complete set of PVT data to one of a set of detailed fluid components, or to a common set of components and pseudo-components; lumping (202) the PVT data from the complete set of PVT data into a pre-defined set of components and pre-defined set of pseudo-components to generate a plurality of equation of state (EoS) models; generating (203) for the PVT samples on the PVT data set an EoS model using a same set of tuning parameters and thereby generating an EoS fluid model fingerprint for the hydrocarbon fluid samples; and associating (204) properties of the hydrocarbon fluid samples with the generated EoS fluid model fingerprint.

FIG. 2

**Description**

**FIELD OF THE INVENTION**

The present invention relates to a computer-implemented method and system for predicting hydrocarbon fluid properties using machine-learning-based models. The present invention further relates to a computer-implemented method and system for generating equations of state (EoS) for a plurality of hydrocarbon fluids using machine-learning-based models.

**BACKGROUND OF THE INVENTION**

Petroleum consists of a complex mixture of hydrocarbons of various molecular weights, plus other organic compounds. The exact molecular composition of petroleum varies widely from formation to formation. The proportion of hydrocarbons in the mixture is highly variable and ranges from as much as 97% by weight in the lighter oils to as little as 50% in the heavier oils and bitumens. The hydrocarbons in petroleum are mostly alkanes (linear or branched), cycloalkanes, aromatic hydrocarbons, or more complicated chemicals like asphaltenes. The other organic compounds in petroleum typically contain carbon dioxide ($CO_2$), nitrogen, oxygen, and sulfur, and trace amounts of metals such as iron, nickel, copper, and vanadium.

Hydrocarbon fluid properties are of importance in the oil and gas industry. They are essential for calculating the amount of the hydrocarbons initially in place, for reservoir simulation and production forecasting as well as for well, completion, pipeline and surface facility design. For measuring hydrocarbon fluid properties, typically, the pressure-volume-temperature (PVT) properties are measured as a function of pressure. Therefore, PVT data for hydrocarbon fluid samples is necessary. This PVT data may be acquired at different location of the production system: e.g. well bottom hole, well tubing head, and at the outlet of the last separator stage. Once acquired, this PVT data is sent to the laboratory for analysis where the above-mentioned properties are measured. Nevertheless, regardless of the number of PVT data for hydrocarbon fluid samples that are acquired and the laboratory measurements that are performed, a thermodynamic model is typically used, such as an equation of state (EoS) model that represents the phase behavior of the petroleum fluid in the reservoir and is used to predict the hydrocarbon fluid properties under the expected range of pressure and temperature covering the life of the reservoir and the whole production system. Once the EoS model is defined, it can be used to compute a wide array of properties of the petroleum fluid of the reservoir, such as gas-oil ratio (GOR) or condensate-gas ratio (CGR), density of each phase, volumetric factors and compressibility, and heat capacity and saturation pressure (bubble or dew point). Thus, the EoS model can be solved to obtain saturation pressure at a given temperature. Moreover, GOR, CGR, phase densities, and volumetric factors are byproducts of the EoS model. Other properties, such as heat capacity or viscosity, can also be derived in conjunction with the information regarding fluid composition. Furthermore, the EoS model can be extended with other reservoir evaluation techniques for compositional simulation of flow and production behavior of the petroleum fluid of the reservoir, as is well known in the art.

To build a typical EoS model from laboratory measurements, a minimum set of measurements are required e.g. compositional properties (mole fractions of the components (e.g. N2, H2S, CO2, C1, C2, C3, C4, C5, C7, C8, etc.) and pseud-components (e.g. C7+, C12+, C20+ and C36+ or any other pseudo-component) as well as the molecular weight of the pseudo-components), constant composition expansion (CCE), constant volume depletion (CVD), differential liberation (DL) and multi-stage separator (MSS).

Building machine-learning-based models based on PVT data is of paramount importance to capture trends and predict fluid models in a very heterogeneous thermodynamic system exhibiting highly non-linear behaviors. PVT data for hydrocarbon fluid samples stored in an oil company database are often not complete and might be missing properties, both black oil and compositional ones. Therefore, it is required to have a clean and structured PVT database complete with all required properties. In the presence of a large set of PVT data for hydrocarbon fluid samples, some of these PVT data may lack part of the fluid properties as they may not have been measured in the laboratory. In some scenarios, the compositional properties in the database are absent as no representative PVT data could be obtained. In this case, the list of measurements is restricted to some properties obtained at stock tank conditions. A large set of existing PVT data may be lacking the PVT granularity of the heavy end (e.g. they may be restricted to C7+). Companies with multiple fields and reservoirs may have an extensive set of EoS built using different PVT data (with their corresponding laboratory analysis). When new PVT data is acquired for a new field or from a specific region of an existing field, is it necessary to measure similarities with existing PVT data of hydrocarbon fluid samples to either map the sample to an existing fluid model or intelligently predict a new one.

Predicting PVT data properties using machine learning or artificial intelligence is known in the art. With respect to predicting compositional properties from other compositional properties, Wang et al. (Wang, K., Zuo, Y. and Jalali, Y., Schlumberger Technology Corp, 2017. *Prediction of Fluid Composition and/or Phase Behavior.* U.S. Patent Application 15/193,519) propose and test machine learning algorithms to predict and/or calculate components mole fractions (CO2, C1, C2, C3, C4, C5 and C6+) as well as C6+ molecular weight from components weight fractions. Wang et al. elaborate two workflows, one starting from weight fractions of CO2, C1, C2, C3, C4, C5 and C6+, predict using machine learning the mole fraction of C6+ and then calculate, using pertinent equations relating molar weight to mole fractions and mass fractions, the molecular weight of C6+ followed by calculating, using pertinent equations relating mole fractions to weight fractions, the mole fractions of CO2, C1, C2, C3, C4, C5, and the other starting from weight fractions of CO2, C1, C2, C3, C4, C5 and C6+, predict using machine learning the mole fractions of CO2, C1, C2, C3, C4, C5 and C6+.

Oil companies with multiple fields and reservoirs may have multiple EoS models built using different PVT data for hydrocarbon fluid samples (with their corresponding laboratory analysis). One or more PVT data sets may be used to build and calibrate an EoS model. These EoS models are typically built to model volumetric properties and phase behavior of one or more reservoirs or even a region in one reservoir in a specific field. EoS models are also necessary to build dynamic models of the subsurface reservoirs, and these models are key drivers in understanding reservoir performance and optimizing field development plans. The representativity of the reservoir with accurate fluid models is a big challenge, costly job, and it requires intensive and rigorous analysis. Multiple challenges are encountered through the process. Different known EoS models are built by different experts without coordination between these experts. The known EoS models are then tuned using different tuning parameters due to the non-uniqueness of the tuning process. Furthermore, different component and pseudo-components grouping/lumping schemes are applied by different experts as, again, the grouping/lumping is not a unique process, by nature.

## SUMMARY OF THE INVENTION

It is therefore an object of the present invention to provide an improved computer-implemented method and system for predicting fluid hydrocarbon fluid properties using machine-learning-based models. It is further an object of the present invention to provide an improved computer-implemented method and system for generating equations of state (EoS) for a plurality of hydrocarbon fluids.

In view of the state of the known technology and in accordance with a first aspect of the present invention, a computer-implemented method for predicting hydrocarbon fluid properties using machine-learning-based models comprises the method steps of: receiving a minimal set of pressure-volume-temperature (PVT) data for hydrocarbon fluid samples from a PVT data base; reading the minimal set of PVT data by a reader module; transforming the minimal set of PVT data into a unified data structure by the reader module; selecting items of the PVT data from the minimal set of PVT data by the reader module; processing the selected items of the PVT data by a correlating module to identify a plurality of correlations in the selected items of the PVT data based on one or more of the fluid properties of the hydrocarbon fluid samples; clustering, using of at least one of a plurality of clustering algorithms, the selected items of the PVT data into a plurality of clusters by a clustering module; and performing machine learning by a machine learning module on ones of the plurality of clusters to predict missing fluid properties in the minimal set of PVT data and thus to obtain a complete set of PVT data.

The computer-implemented method in accordance to the first aspect of the present invention provides a systematic methodology to complete PVT data for properties for hydrocarbon fluid in a consistent manner.

In another aspect, the step of performing machine learning by the machine learning module of the computer-implemented method further comprises the step of predicting of fluid properties for future sets of PVT data for the hydrocarbon fluid samples.

In another aspect, the computer-implemented method comprises the step of plotting the machine learning predictions by the machine learning module.

In another aspect, the computer-implemented method comprises the step of comparing the identified plurality of correlation results with the machine learning predictions.

In another aspect, the step of clustering of the computer-implemented method further comprises the step of completing fluid composition including C12+, C20+ and C36+ mole fraction and molecular weight and/or completing black oil properties, including, in the following order: solution gas oil ratio (GOR), BO@Psat, and saturation pressure (Psat).

In view of the state of the known technology and in accordance with a second aspect of the present invention, a system for predicting hydrocarbon fluid properties using machine-learning-based models comprises a pressure-volume-temperature (PVT) data base, a reader module, a correlating module, a clustering module, and a machine learning module. The pressure-volume-temperature (PVT) data base provides a minimal set of pressure-volume-temperature (PVT) data for hydrocarbon fluid samples. The reader module reads in a minimal set of PVT data, wherein the reader module is configured to transform the minimal set of PVT data into a unified data structure, and wherein the reader module is configured to select items of the PVT data from the minimal set of PVT data. The correlating module for processes the selected items of the PVT data to identify a plurality of correlations in the selected items of the PVT data based on one or more of the fluid properties of the hydrocarbon fluid samples. The clustering module clusters, using of at least one of a plurality of clustering algorithms, the selected items of the PVT data into a plurality of clusters. The machine learning module performs machine learning on ones of the plurality of clusters to predict missing fluid properties in the minimal set of PVT data and thus to obtain a complete set of PVT data.

In view of the state of the known technology and in accordance with a third aspect of the present invention, a computer-implemented method for generating equations of state (EoS) for a plurality of hydrocarbon fluids comprises the method steps of: delumping pressure-volume-temperature (PVT) data for hydrocarbon fluid samples from a complete set of PVT data to one of a set of detailed fluid components, or to a common set of components and pseudo-components; lumping the PVT data from the complete set of PVT data into a pre-defined set of components and pre-defined set of pseudo-components to generate a plurality of equation of state (EoS) models; generating for the PVT samples on the PVT data set an EoS model using a same set of tuning parameters and thereby generating an EoS fluid model fingerprint for the hydrocarbon fluid samples; and associating properties of the hydrocarbon fluid samples with the generated EoS fluid model fingerprint.

The computer-implemented method in accordance to the third aspect of the present invention leverages advancement in data sciences to unlock hidden information, patterns and relationships from massive volume and variety of reservoir hydrocarbon fluid data. The developed AI based algorithms can be used to consistently validate the hydrocarbon fluid data (called, interchangeably PVT data), cluster the data and accordingly build machine learning based fluid models, i.e. equation of state (EoS), for different fields and reservoirs based on massive reservoir fluid information. The computer-implemented method in accordance to the second aspect of the present invention ensures reservoir models are managed with the highest confidence and accuracy with quality fluid data, maximizing returns while minimizing costs associated with EoS models.

In another aspect, the computer-implemented method comprises the step of training machine learning models to predict an EoS fluid model fingerprint for new hydrocarbon fluid samples.

In another aspect, the received or provided minimal set of PVT data for hydrocarbon fluid samples comprises black oil properties and compositional properties, wherein the black oil properties comprise at least one of reservoir temperature, solution gas-oil ratio, oil API gravity, gas gravity, dead oil viscosity, saturation pressure, saturated bubble point oil formation factor at saturation pressure, fluid density at reservoir conditions or any other black oil property, fluid compressibility at reservoir conditions, viscosity at reservoir conditions, fluid density at reservoir conditions or any other black oil property, and wherein the compositional properties comprise at least one of mole fractions of the components, in particular N2, H2S, CO2, C1, C2, C3, C4, C5, C7, C8, and pseud-components, in particular C7+, C12+, C20+ and C36+ or any other pseudo-component as well as the molecular weight of the pseudo-components.

In another aspect, the step of associating of the computer-implemented method further comprises the step of clustering the selected items of the PVT data into a plurality of clusters for performing machine learning on each one of the plurality of clusters.

In another aspect, the computer-implemented method comprises the step of comparing the results from clustering of the selected items of the PVT data with the plurality of equations of state (EoS) models and with heatmaps applying EoS models on the selected items of the PVT data.

In another aspect, the step of clustering of the selected items of the PVT data further comprises the step of identifying of clusters to which PVT data belong.

In view of the state of the known technology and in accordance with a fourth aspect of the present invention, a system for generating equations of state (EoS) for a plurality of hydrocarbon fluids comprises a first module, a second module, a third module and a fourth module. The first module delumps pressure-volume-temperature (PVT) data for hydrocarbon fluid samples from a complete set of PVT data to one of a set of detailed fluid components, or to a common set of components and pseudo-components. The second module lumps the PVT data for hydrocarbon fluid sample into a pre-defined set of components and pre-defined set of pseudo-components to generate a plurality of equation of state (EoS) models. The third module generates for the hydrocarbon fluid samples in a PVT data base an EoS model using a same set of tuning parameters and thereby generating an EoS fluid model fingerprint for the hydrocarbon fluid samples. The fourth module associates properties of the hydrocarbon fluid samples with the generated EoS fluid model fingerprint.

The present invention has a great impact on the way fluid modeling is performed. The present invention provides greater insights and better understanding of oil and gas fields and reservoirs. Furthermore, the results of the methods presented in the present invention can be scaled in very dynamic way to other fields in other regions. The present methods are based on a smart technology that adapts to the physical variation across fluid systems and perform fluid modeling with the highest confidence and accuracy. The technology is extendable and subject to integration with other subsurface domains as a smart fluid laboratory.

Also, other objects, features, aspects and advantages of the disclosed method will become apparent to those skilled in the art from the following detailed description.

## BRIEF DESCRIPTION OF THE DRAWINGS

The invention will now be described on the basis of figures. It will be understood that the embodiments and aspects of the invention described in the figures are only examples and do not limit the protective scope of the claims in any way. The invention is defined by the claims and their equivalents. It will be understood that features of one aspect or embodiment of the invention can be combined with a feature of a different aspect or aspects of other embodiments of the invention. The present invention becomes more obvious when reading the following detailed descriptions of some examples as part of the disclosure under consideration of the enclosed drawings. Referring now to the attached drawings which form a part of this disclosure:

Fig. 1A is a schematic illustration of Fields and Reservoirs and PVT data spread over the two reservoirs, in particular of three field illustrating two reservoirs.

Fig. 1B is a flow diagram of a computer-implemented method for predicting hydrocarbon fluid properties using machine-learning-based models in accordance to a first aspect of the present invention.

Fig. 2 is a schematic illustration of the overall view of the workflow diagram of the computer-implemented method for predicting hydrocarbon fluid properties using machine-learning-based models in accordance to the first aspect of the present invention.

Fig. 3 is a schematic illustration of a system for predicting hydrocarbon fluid properties using machine-learning-based models in accordance to a second aspect of the present invention.

Fig. 4 is a workflow diagram of the data reader module.

Fig. 5 is a workflow diagram of the clustering module.

Fig. 6 is a workflow diagram of the correlating module.

Fig. 7 is a workflow diagram of the machine learning module.

Fig. 8 is a table of data ingestion and preparation for machine learning.

Fig. 9 is a distribution over different fields of the data set used in the implementation and validation process, in particular the data belongs to a total of 65 fields.

Fig. 10 is a distribution over different reservoirs of the data set used in the implementation and validation process, in particular the data belongs to a total of 64 reservoirs.

Fig. 11 is a PVT Data set used in the implementation and validation process, in particular data statistics and black oil properties.

Fig. 12 is a PVT Data set used in the implementation and validation process, particular data statistics, compositional properties, and heavy fractions.

Fig. 13 is a PVT Data set used in the implementation and validation process, in particular data statistics, compositional properties, light and intermediate components.

Figs. 14A and 14B are EDA Results applied to the PVT Data set used in the implementation and validation process, in particular Fig. 14A for black oil properties and Fig. 14B for black oil and compositional properties.

Figs. 15A and 15B are EDA Results, in particular black oil properties, in particular shows Fig. 15A the worst correlations and Fig. 15B the best correlations. The properties included in the EDA of Figs. 15A and 15B are illustrated in Fig. 14A.

Figs. 16A and 16B are EDA Results, in particular compositional properties, in particular shows Fig. 16A the worst correlations and Fig. 16B the best correlations. The properties included in the EDA of Figs. 16A and 16B are illustrated in Fig. 14B.

Fig. 17 is a flow diagram of a computer-implemented method for generating equations of state (EoS) for a plurality of hydrocarbon fluids in accordance to a second aspect of the present invention.

Fig. 18 is a schematic illustration of a system for for generating equations of state (EoS) for a plurality of hydrocarbon fluids in accordance to a fourth aspect of the present invention.

Fig. 19 is an overview diagram of the hydrocarbon fluid properties and EoS prediction using machine learning subject of the present invention.

Fig. 20 is an overview of the machine-learning based EoS workflow.

Fig. 21 illustrates the lumping scheme used in the process of creating the EoS model using regression.

Fig. 22 is a Heatmap resulting from Exploratory Data analysis (EDA) preceding ML to predict the EoS Parameters. TCRIT (C20P) and PCRIT (C20P) dependency on different BO and Compositional parameters. Results: Parameters to be used in ML are: Mole Fraction of C1, C6, C7+, C12+, C20+, C36+, MW of C7+, C12+, C36+, Psat, GOR, Bo@Psat, Density@Psat, API Gravity and Tres. Furthermore, chain rule is used: PCRIT of C20P is added to inputs to predict TCRIT of C20P.

Fig. 23A and 23B are an illustration of the predicted EoS parameters using ML when using Pcrit and Tcrit of C20P of C20P as regression parameters. The figures depicts Pcrit (Fig. 23A) and Tcrit (Fig. 23B) of C20P from ML vs. those obtained using conventional Regression.

Fig. 24 illustrates example results of machine-learning based EoS - Sample 1, Field F1, Reservoir R1. The figure shows actual data, initial guess of EoS model without any regression, Conventional regression based EoS and ML based EoS.

Fig. 25 illustrates other example results of machine-learning based EoS - Sample 2, Field F2, Reservoir R2. The figure shows actual data, initial guess of EoS model without any regression, Conventional regression based EoS and ML based EoS.

Fig. 26 illustrates other example results of machine-learning based EoS - Sample 3, Field F3, Reservoir R3. The figure shows actual data, initial guess of EoS model without any regression, Conventional regression based EoS and ML based EoS.

Fig. 27 illustrates machine-learning Based EoS - Error Analysis; Train + Test dataset. The EoS parameters resulting from ML are used in a PVT modeling tool to reproduce Laboratory experiments (CCE, DL and MSS). The figure shows the cumulative percentage error (EoS vs. Data) from conventional regression based EoS and ML based EoS.

Fig. 28 illustrates machine-learning Based EoS - Error Analysis; Train + Test dataset. The EoS parameters resulting from ML are used in a PVT modeling tool to reproduce Laboratory experiments (CCE, DL and MSS). The figure shows the percentage error (EoS vs. Data) from conventional regression based EoS and ML based EoS for a selected set of fluid properties resulting from the above-mentioned experiments.

## DETAILED DESCRIPTION OF THE INVENTION

Selected embodiments are now described with reference to the drawings. It is apparent from this disclosure to a person skilled in the art of generic fluid hydrocarbon fluid properties that the following description of embodiments is provided for illustrative purposes only and is not intended to limit the invention defined by the claims attached and their equivalents.

The following nomenclature is used with respect to the present invention:

- MW = Molecular Weight

- N_Samples = Number of PVT Samples (a total of 1700 PVT samples)

- DETAILED COMPOSTION: detailed set of components = CO2, H2S, N2, C1, C2, C3, iC4, nC4, iC5, nC5, C6, C7, ..., C35, C36+

- EXPERIMENTS = Selected set of experiments. Set includes CCE, CVD, DL and MSS. CCE may be a good candidate as a proof of concept.

Note: An entire experiment can be used. Alternatively, a selected set of properties from an experiment can be used. Example: Psat, Bo@Psat, GOR, API, etc.

- SAMPLES = Selected set of PVT samples with their associated EXPERIMENTS. This may be the complete set of samples (N_Samples), all oil samples or the set validated oil samples used to build the EoS models. In this milestone, focus would be on the Validated Oil Samples.

• EoS MODELS = Set of EoS models to be used in Tasks 2 and 3. The total number is ~20.
• TUNING PARAMETERS = Set of EoS parameters (P1, P2, P3, ...., PN) to be tuned / modified, the range of uncertainty of these parameters and the associated weights.
• FLUID PROPERTIES = PVT properties as set out below denote a multitude of hydrocarbon fluid properties under different temperatures and pressures, from the reservoir (at reservoir pressure and temperature) to the last separator stage; that is stock tank oil and gas at standard conditions. Properties are typically gathered under two categories:
Compositional properties: These are mainly the mole fractions of the components (e.g. N2, H2S, CO2, C1, C2, C3, C4, C5, C7, C8, etc.) and pseud-components (e.g. C7+, C12+, C20+ and C36+ or any other pseudo-component) as well as the molecular weight of the pseudo-components. Note that the choice of pseudo-components used for the purpose of this project is not unique. The proposed algorithm work for any choice of pseudo-components.
Black Oil Properties. Examples include, but not restricted to, properties presented in the below tables

| $T\_R$ | Reservoir Temperature |
|---|---|
| $R\_s$ | Solution gas-oil ratio |
| $\gamma\_API$ | Oil API gravity |
| $\gamma\_g$ | Gas gravity |
| $\mu\_od$ | Dead oil viscosity |
| $Psat$ | Saturation pressure |
| $Bob @ Psat$ | Saturated bubble point oil formation factor at saturation pressure |
| $\rho\_psat$ | Fluid density at reservoir conditions |
| $C\_psat$ | Fluid compressibility at reservoir conditions |
| $\mu\_psat$ | Viscosity at reservoir conditions |
| $\rho\_res$ | Fluid density at reservoir conditions |

• FIELD = An accumulation, pool or group of pools of oil/gas in the subsurface. An oil/gas field consists of reservoirs in a shape that will trap hydrocarbons and that are covered by an impermeable or sealing rock. Typically, industry professionals use the term with an implied assumption of economic size.
• RESERVOIR = A subsurface body of rock having sufficient porosity and permeability to store and transmit fluids. Sedimentary rocks are the most common reservoir rocks because they have more porosity than most igneous and metamorphic rocks and form under temperature conditions at which hydrocarbons can be preserved.

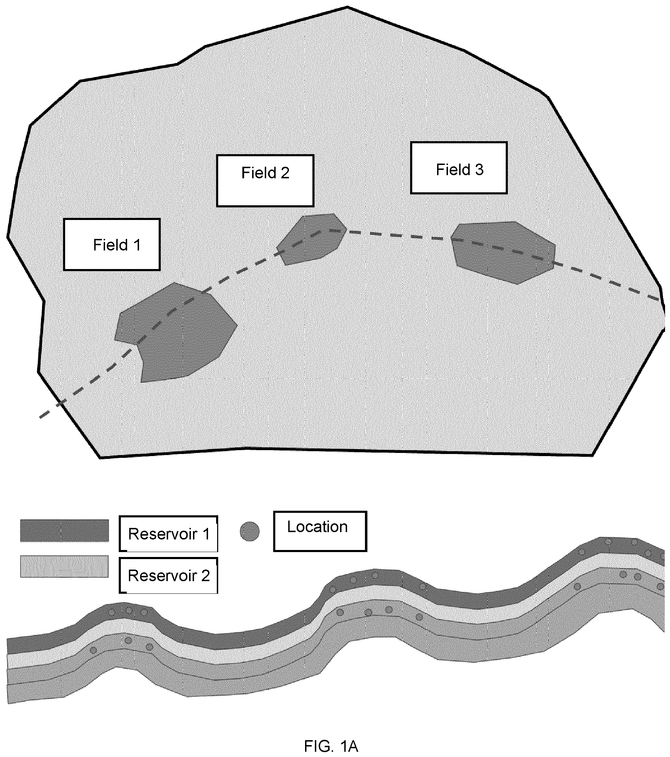

Fig. 1A is a schematic illustration of fields and reservoirs and PVT data spread over the two reservoirs, in particular of three fields illustrating two reservoirs.

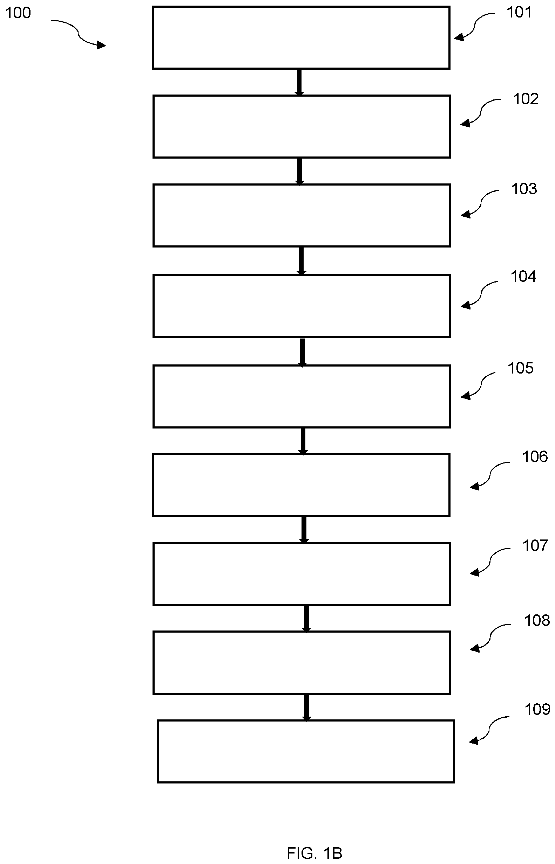

Fig. 1B is a flow diagram of a computer-implemented method for predicting hydrocarbon fluid properties using machine-learning-based models in accordance to a first aspect of the present invention. The computer-implemented method 100 for predicting hydrocarbon fluid properties using machine-learning-based models comprises the step of receiving 101 a minimal set of pressure-volume-temperature (PVT) data for hydrocarbon fluid samples from a PVT data base 301. The method 100 further comprises the step of reading 102 the minimal set of PVT data by a reader module 302. The method 100 comprises the step of transforming 103 the minimal set of PVT data into a unified data structure by the reader module 302. The method 100 further comprises the step of selecting 104 items of the PVT data from the minimal set of PVT data by the reader module 302. The method 100 even further comprises the step of processing 105 the selected items of the PVT data by a correlating module 303 to identify a plurality of correlations in the selected items of the PVT data based on one or more of the fluid properties of the hydrocarbon fluid samples. The method 100 further comprises the step of data clustering 106, using of at least one of a plurality of clustering algorithms, the selected items of the PVT data into a plurality of clusters by a clustering module 304. The method 100 comprises the step of performing 107 machine learning by a machine learning module 305 on ones of the plurality of clusters to predict missing fluid properties in the minimal set of PVT data and thus to obtain a complete set of PVT data.
The minimal set of PVT data for hydrocarbon fluid samples comprises black oil properties and compositional properties, wherein the black oil properties comprise at least one of reservoir temperature, solution gas-oil ratio, oil API gravity, gas gravity, dead oil viscosity, saturation pressure, saturated bubble point oil formation factor at saturation pressure, fluid density at reservoir conditions, fluid compressibility at reservoir conditions, viscosity at reservoir conditions, fluid density at reservoir conditions, and wherein the compositional properties comprise at least one of mole fractions of the components, in particular N2, H2S, CO2, C1, C2, C3, C4, C5, C7, C8, and pseud-components, in particular C7+, C12+, C20+ and C36+ or any other pseudo-component as well as the molecular weight of the pseudo-components.

The data clustering step 106 of method 100 is applied prior to the step of performing 107 machine learning by a machine learning module 305. The data clustering step 106 is used to categorize the PVT data into families based on their collective behavior of the different features of the PVT data and, hence, improve the quality of the PVT samples properties prediction using machine learning.

The method 100 further comprises that the step of performing 107 machine learning by the machine learning module 305 further comprises the step of predicting of fluid properties for future sets of PVT data for the hydrocarbon fluid samples. The method 100 further comprises the step of plotting 108 the machine learning predictions by the machine learning module 305. The method 100 further comprises the step of comparing 109 the identified plurality of correlation results with the machine learning predictions.

The method 100 according to the first aspect of the present invention uses algorithms to predict in series, starting from a minimal set of PVT data (black oil / and compositional) to obtain a complete set of PVT data through method 100 that predicts and uses highest correlating properties first.

The method 100 further comprises that the step of the data clustering 106 further comprises the step of completing fluid composition including C12+, C20+ and C36+ mole fraction and molecular weight and/or completing black oil properties including, in the following order: solution gas oil ratio (GOR), BO@Psat, and saturation pressure (Psat). Thereby, it is possible to learn from existing data and "automatically" complete missing PVT data in every PVT sample using a step-by-step process. The order through which PVT data is completed is set out in the following order and relies on benefiting from the existing PVT data to predict missing PVT data starting from the highest correlating PVT data to the lowest. The following terminology is used:

| | |
|---|---|
| $Prop_{in}$ | Input properties |
| $Prop_{out}$ | Output properties |
| $ML_{error\_train}$ | Calculated error resulting from the Training phase of a given ML method |
| $ML_{error\_test}$ | Calculated error resulting from the Testing phase of a given ML method |
| $N_{pi}$ | Number of samples with the complete set of $Prop_{in}$ |
| $N_{pi\_train}$ | Number of samples with the complete set of $Prop_{in}$ to be used in the Training phase of the ML |
| $N_{pi\_test}$ | Number of samples with the complete set of $Prop_{in}$ to be used in the Testing phase of the ML $$N_{pi\ test} = N_{pi} - N_{pi\_train}$$ |
| $N_{po}$ | Number of samples for which $Prop_{out}$ to be predicted |
| $N_{c\_total}$ | Total number of samples with compositional information |
| $N_{c_{complete_{C7+}}}$ | Number of samples with complete C7+ |
| $N_{c_{complete_{C12+}}}$ | Number of samples with complete C7+ and C12+ |
| $N_{c_{complete_{C20+}}}$ | Number of samples with complete C7+, C12+ and C20+ |
| $N_{c\_complete}$ | Number of samples with complete C7+, C12+, C20+ and C36+ |
| $N_{c_{incomplete_{C12+}}}$ | Number of samples with incomplete C12+ |
| $N_{c_{incomplete_{C20+}}}$ | Number of samples with incomplete C20+ |
| $N_{c_{complete}}$ | Number of samples with incomplete C36+ |
| $N_{c_{incomplete}}$ | Number of samples with incomplete compositional data $$N_{c_{incomplete}} = N_{c\_total} - N_{c\_complete}$$ |

The output properties $Prop_{out}$ are predicted using the most optimal machine learning method. In particular, as an Input: $N\_pi$ samples, $N\_po$ samples and $Prop_{out}$ are used to predict for an output: Complete $Prop_{out}$ for $N\_po$ samples.

The flowing algorithm is used to predict any missing property $Prop_{out}$:

$$ML_{error\_min} = \text{big\_number}$$
For every ML method
Train to predict $Prop_{out}$ using $N\_(pi\_train)$ samples
Test using $N\_(pi\_test)$ samples to test ML's capability to predict $Prop_{out}$
Calculate $ML_{error\_test}$: if $ML_{error\_test} < ML_{error\_min}$ ➜ ML_best = ML
Predict $Prop_{out}$ $(N\_po)$ using ML_best

In one aspect of the present invention, the PVT data can be completed without the data clustering step 106, however this is not limiting the present invention. To complete composition data without the data clustering step 106, as an input all samples with compositional data can be used, in particular, mole fractions of N2, H2S, CO2, C1, C2, C3, C4, C5, C6 and C7+ as well as C7+ MW. The output are samples with completed C12+, C20+ and C36+ mole fraction and molecular weight (for the specific cluster).

The flowing algorithm is used to complete composition data:

ML_optimal (Mole Fraction C12+)
Input: samples with complete C7+ and C12+ Mole Fractions and MW

Output: Completed C12+ Mole Fraction for all samples
ML_optimal (MW C12+)

Input: samples with complete C7+ and C12+ Mole Fractions and MW

Output: Completed C12+ MW for all samples
ML_optimal (Mole Fraction C20+)

Input: samples with complete C7+, C12+ and C20+ Mole Fractions and MW

Output: Completed C20+ Mole Fraction for all samples
ML_optimal (MW C20+)

Input: samples with complete C7+, C12+ and C20+ Mole Fractions and MW

Output: Completed C20+ MW for all samples
ML_optimal (Mole Fraction C36+)

Input: samples with complete C7+, C12+, C20+ and C36+ Mole Fractions and MW

Output: Completed C36+ Mole Fraction for all samples
ML_optimal (MW C36+)

Input: samples with complete C7+, C12+, C20+ and C36+ Mole Fractions and MW

Output: Completed C36+ MW for all samples

To complete black oil data, as an input already completed C12+, C20+ and C36+ mole fractions and MW can be used. The output are samples with completed black oil properties.

The flowing algorithm is used to complete black oil data:

ML_optimal (GOR)
Input: All samples (Compositional); some missing GOR.
At Output: All samples have GOR

ML_optimal (BO_Psat)
Input: All samples (Compositional and GOR); some missing BO_Psat
At Output: All samples have GOR and BO_Psat
ML_optimal (Psat)
Input: All samples (Compositional, GOR and BO_Psat); some missing Psat
At Output: All samples have GOR, BO_Psat and Psat

In another aspect of the present invention, the PVT data can be completed with the data clustering step 106, however this is not limiting the present invention. To complete composition data with the data from the data clustering step 106, as an input all samples with compositional data can be used, in particular, mole fractions of N2, H2S, CO2, C1, C2, C3, C4, C5, C6 and C7+ as well as C7+ MW. As an output, every sample will be associated with one of the clusters identified in the data clustering step 106.

The flowing algorithm is used to complete composition and black oil data:

```
For every Clustering Method (CM)
                    Cluster_Data
                    For every cluster
                          Complete_Composition_Data of samples belonging to that
               cluster
                          Complete_BO_Properties of samples belonging to that cluster
                          Calculate cumulative ML_error_min (from all clusters and all
               properties)
                          Select the clustering method that results on the lowest
               cumulative ML_error_min
```

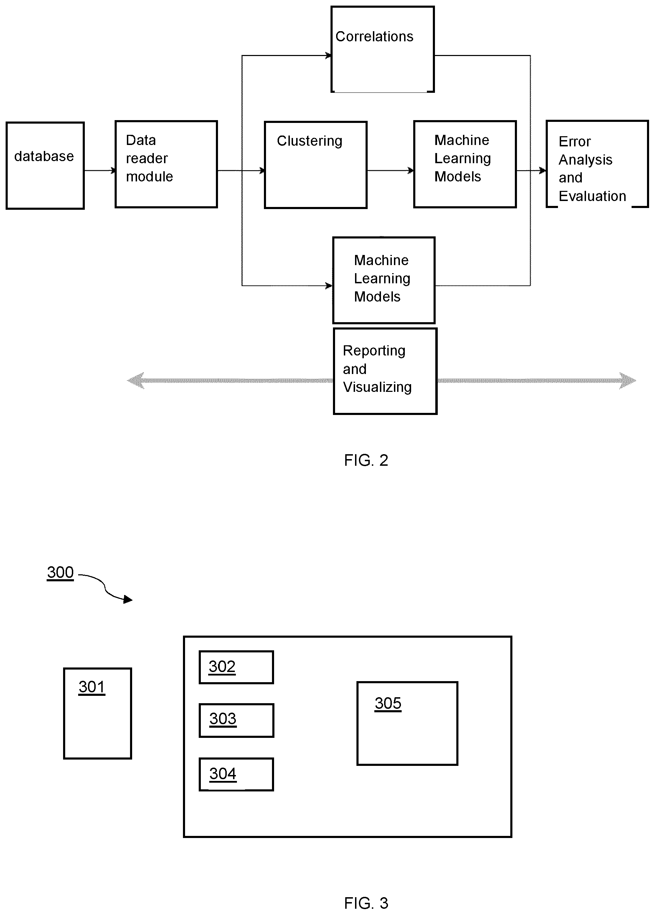

Fig. 2 is a schematic illustration of the overall view of the workflow of the computer-implemented method for predicting hydrocarbon fluid properties using machine-learning-based models in accordance to the first aspect of the present invention.

PVT data set is parsed into a standard format and passed to later components in the data reader module 305. The step of data clustering 106 divides PVT data from the PVT data set into groups such that samples in one cluster are likely to be similar. This unravels hidden insights and facilitates learning and improve performance. Empirical correlations available in the literature can be applied to provide reference results (in terms of predictive capability) to the final machine learning results. The step of machine learning can be performed on the whole PVT data set at once. More importantly, machine learning can be performed on each of the clusters to predict missing PVT properties and prepare for predicting PVT properties for future PVT data. As can be seen in Fig. 2, analysis is performed to compare machine learning predictions with empirical correlations. Further, logging and reporting (e.g. Excel, PDF and JPEG) is taking place at every step.

Fig. 3 is a schematic illustration of a system 300 for predicting hydrocarbon fluid properties using machine-learning-based models in accordance to a second aspect of the present invention. Fig. 3 shows the system 300 for predicting hydrocarbon fluid properties using machine-learning-based models, wherein the system 300 comprises a pressure-volume-temperature (PVT) data base 301, a reader module 302, a correlating module 303, a clustering module 304, and a machine learning module 305. The pressure-volume-temperature (PVT) data base 301 provides a minimal set of pressure-volume-temperature (PVT) data for hydrocarbon fluid samples. The reader module 302 reads in a minimal set of PVT data, wherein the reader module 305 is configured to transform the minimal set of PVT data into a unified data structure, and wherein the reader module 30 is configured to select items of the PVT data from the minimal set of PVT data. The correlating module 303 processes the selected items of the PVT data to identify a plurality of correlations in the selected items of the PVT data based on one or more of the fluid properties of the hydrocarbon fluid samples. The clustering module 304 clusters, using of at least one of a plurality of the above clustering algorithms, the selected items of the PVT data into a plurality of clusters. The machine learning module 305 performs machine learning on ones of the plurality of clusters to predict missing fluid properties in the minimal set of PVT data and thus to obtain a complete set of PVT data.

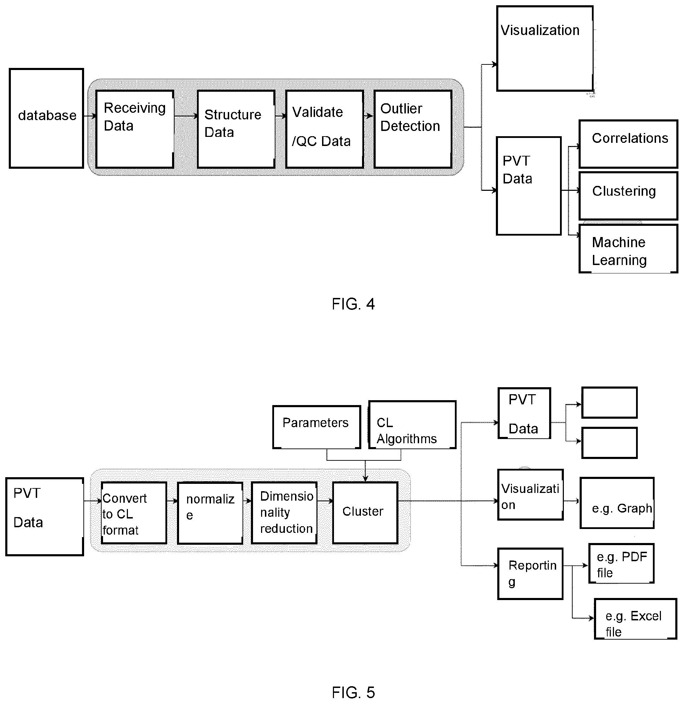

Fig. 4 shows a workflow of the data reader module 302. PVT Data is provided from the PVT data base 301 (e.g. in excel format). The desired PVT properties are parsed and extracted. The PVT data is then transformed into a unified data structure to undergo further processing. A quality check is performed to account for out of range properties (e.g. negative values), duplicate samples, etc., wherein out of range values (e.g., API_gravity 0 → 100) are considered outliers. The statistics are visualized and reported. The validated PVT data will proceed to further components such as clustering, machine learning, correlations, etc.

Fig. 5 shows a workflow of the clustering module 303. The required PVT data structure is received from the data reader module 302. The received data is converted into a custom format on which the step of data clustering 106 can operate. This custom format is substantially a 2D matrix in which each row represents a sample, and properties of the sample are represented by columns. PVT properties with different ranges can screw clustering models since properties having high ranges are considered of high importance. Therefore, normalizing data to have zero mean and unit variance is necessary. In high dimensional spaces, the step of data clustering 106 may not work well, hence, dimensionality reduction might be useful. The step of data clustering 106 will be performed using several clustering algorithms available in the literature, as described above. The clustering results and comparisons (e.g., properties before versus properties after clustering) are visualized. Figures are reported in e.g. a PDF, whereas resulting tables are reported in e.g. an excel that can be later imported in e.g. spotfire. The PVT data structure is updated with its cluster ID and passed to other components such as the machine learning module 305.

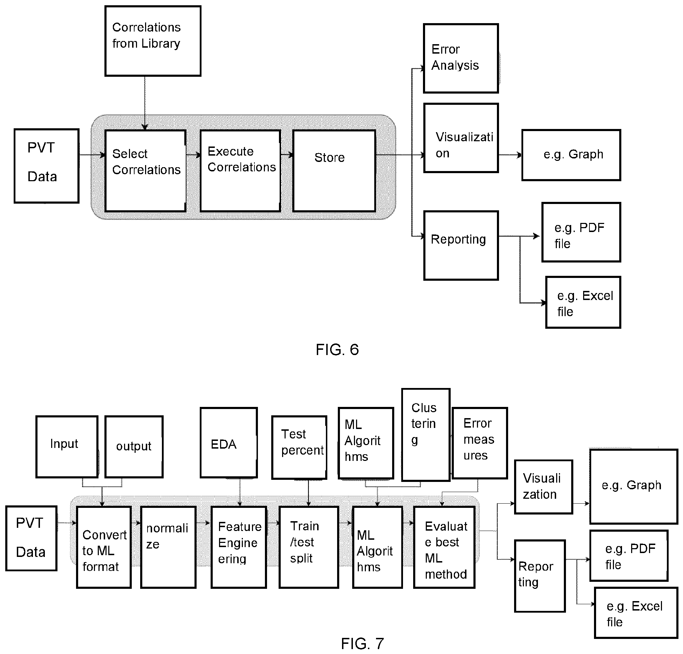

Fig. 6 shows a workflow diagram of the correlating module 303. The required PVT data structure is received from the data reader module 302. A plurality of correlations found in the literature are processed for particular properties (Psat, Bo@Psat and Dead oil viscosity).

The following correlations are processed in the present invention: Psat

- Standing (Standing 1947)
- Al Marhoun (Al-Marhoun 1988)
- Vazquez and Beggs (Vazquez and Beggs 1980)
- Kartoatmodjo and Schmidt (Kartoatmodjo and Schmidt 1994)
- Dokla and Osman (Dokla and Osman 1992)
- Petrosky Jr. Farshad (Petrosky Jr and Farshad 1993)
- Al Shammasi (Al-Shammasi 2001)
- Dindoruk & Christman (Dindoruk and Christman 2001)
- Khamehchi et al. (Khamehchi, Rashidi et al. 2009)
- Arabloo et al. (Arabloo, Amooie et al. 2014)
- Jarrahian et al. (Jarrahian, Moghadasi et al. 2015)


Bo@Psat

- Standing (Standing 1947)
- Al Marhoun (Al-Marhoun 1988)
- Vazquez and Beggs (Vazquez and Beggs 1980)
- Kartoatmodjo and Schmidt (Kartoatmodjo and Schmidt 1994)
- Dokla and Osman (Dokla and Osman 1992)
- Petrosky Jr.F Arshad (Petrosky Jr and Farshad 1993)
- Omar and Todd (Omar and Todd 1993)
- Al Mehaideb (Almehaideb 1997)
- Al Shammasi (Al-Shammasi 2001)
- Dindoruk and Christman (Dindoruk and Christman 2001)
- Ikiensikimama and Ajienka (Ikiensikimama and Ajienka 2012)
- Arabloo et al. (Arabloo, Amooie et al. 2014)


Dead oil viscosity

- Beggs and Robinson (Beggs and Robinson 1975)
- Glaso (Glaso 1980)
- Dindoruk and Christman (Dindoruk and Christman 2001)
- Naseri et al. (Naseri, Nikazar et al. 2005)
- Kartoatmodjo and Schmidt (Kartoatmodjo and Schmidt 1994)
- Petrosky Jr. Farshad (Petrosky Jr and Farshad 1993)
- Labedi (Labedi 1992)


As can be seen in Fig. 6, the selected correlations are processed and the resulting values from each of the selected correlations are for example returned in a vector format. The resulting vectors are stored in a table format for every PVT data and for every property. At a later stage, the obtained correlation values will be compared with the machine learning results. Errors are calculated using the resulting values from the empirical correlations and the real values. The errors are visualized graphically. The figures are reported e.g. in a PDF, whereas resulting tables are reported in e.g. an excel format that can be later imported to visualization tools (e.g. in spotfire).

Fig. 7 shows a workflow of the machine learning module 305. The required PVT data structure is received from data reader module 302. The received PVT data is converted into a custom format that machine learning models can operate on, e.g. the afore-mentioned 2D matrix in which each row represents a sample, and properties are represented by columns. The properties with different ranges mess up the machine learning models, as mentioned above and therefore, normalizing PVT data to have zero mean and unit variance is necessary. For example, Exploratory Data Analysis (EDA) can be utilized to conclude PVT properties correlations and which PVT properties affect a particular output the most. To ensure model generalization, PVT data is divided into two parts, training data and evaluation data. Several machine learning methods are trained. If clustering preceded machine learning, training is applied on every individual cluster. The best machine learning model is chosen based on minimum error (e.g., RMSE) on the test (hidden) data. The machine learning predictions are plotted and compared with correlations. Further, figures can be reported e.g. in a PDF, whereas resulting tables can be reported reported e.g. in an excel that can be later imported e.g. in spotfire.

The following list of machine learning can be processed with the present invention, however, the present invention is not limited thereby.

LR = Linear Regression;

SVR = Support Vector Regression;

KN = K Neighbors Regression;

RT = Regression Tree;

ET = Extra Tree;

RF = Random Forest;

GB = Gradient Boosting;

MLP = Multi-layer Perceptron;

Bagging;
Adaboost; and
Ensemble.

For achieving the expected result in the present invention, the following steps are proceeded. In the first step the code design is ingested, whereas the PVT data base 301 is read. The PVT data base 301 is structured for example as multiple excel files as follows: PVT Project: containing information about the project of which the fluid sample was taken such as the project's name, the year, the laboratory in which the experiment was done, the well from which the sample was taken, etc.; PVT Black Oil Properties: containing the black oil properties for each fluid sample such as the oil gravity API, reservoir temperature, pressure, bubble point pressure, oil formation volume factor at the bubble point pressure, etc.; Well Coordinates: containing entries of all the wells with the X and Y coordinates of each; and Compositions: containing the molecular composition for each fluid sample for the three sample types which are the "Reservoir Field", "Evolved Gas", and "Stock-Tank Oil". The molecular composition of the heavy components is lumped.

In order to have the complete PVT data for each fluid sample, PVT data from the different excel files are merged and linked to each other using the project ID which is unique for each project. Therefore, the PVT project and PVT Black oil properties are merged using the project ID as unique key for the sample. To add the X and Y coordinates to each fluid sample, each sample is linked to the Well Coordinates excel file with its well name. Then, the coordinates of that well are associated with the sample. The composition of each sample is also linked with previous data using the project ID. The mole fractions of C7+, C12+, C20+, and C36+ are calculated as follows: Mole fraction of C7+ = mole fraction of M-C-C5 + ... + mole fraction of C36; Mole fraction of C12+ = mole fraction of C12 + ... + mole fraction of C36; Mole fraction of C20+ = mole fraction of C20 + ... + mole fraction of C36; Mole fraction of C36+ = mole fraction of C36. However, heavy components are lumped, and the heavy component (Plus Fraction) is indicated in the PVT project excel file. Therefore, when the heavy component is reached, all other mole fractions should be zero. The molecular weights of C7+, C12+, C20+, and C36+ are also calculated by performing several intermediate calculations and using the previously calculated mole fractions.

In the second step data a screening and quality check (QC) is applied. The screening and quality check mechanism is applied to better understand the PVT data and to ensure that the PVT data does not contain any anomalies. This step is very crucial before the PVT data is used by the clustering and machine learning algorithms. PVT data identified with anomalies are flagged so that they are not used in the clustering module 303 and machine learning modules 305. The steps of screening and QC comprises the following: Identifying anomalies in the input PVT data i.e. unphysical values; Flagging PVT data and preparing data structure with proper indicators on complete and missing properties from each of the PVT data. This is a very important step before passing the information to the clustering and machine learning modules. This is also a very important step to be able to complete the information once machine learning models are built.

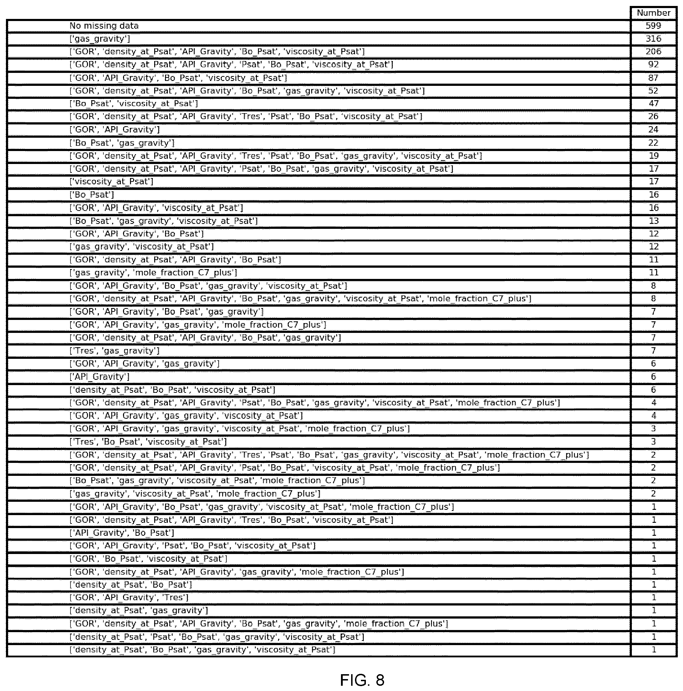

The results of the screening and QC of the PVT data base 301 are the following: The total number of PVT data is for example 1711. With respect to the black oil properties, 593 out of 568 samples have a complete BO set of properties with regard to the black oil properties used in the present invention, as can be seen in Fig. 8 (table). The number of samples will increase or shrink depending how many properties are considered in the machine learning module 305. With respect to the compositional information, the number of samples with valid composition to be used in machine learning module 305 is 1599. A missing heavy fraction, however, does not mean that the heavy fraction does not exist, moreover a "lumped" sample including the missing heavy fraction is existing. With respect to the dynamic data ingestion, every step uses the maximum number of samples. For example, when machine learning is used to predict Psat from "Composition" properties only, all the samples that have values for Psat and Composition should be used. These samples may not have Bo or other Black Oil properties. Accordingly, the step during which samples to be tagged complete / incomplete is a "dynamic" tag rather than a "static" tag. For machine learning, Clustering or EDA, this tagging step is performed once "Run" has been clicked. The general rules for samples with Mole Fraction C7+, C12+, C20+ or C36+ = Zero are as follows: Mole Fraction of C7+, C12+, C20+ or C36+ are typically > 0 for Hydrocarbon Liquid samples; Mole Fraction of C36+ is typically = 0 for Hydrocarbon Vapor (gas) samples. Mole Fraction of C20+ is typically = 0 for Hydrocarbon Vapor (gas) samples unless we have a gas condensate reservoir; Mole Fraction of C7+ is typically > 0 for Hydrocarbon Vapor (gas) samples unless we have dry gas (predominantly methane); Molecular Weight is » 0. However, it should be "meaningless", when the mole fraction = 0. It should not be used in this case. Therefore, mole fraction of C36+ may be 0. It is a valid value (given the above). However, in this case, the Molecular Weight of C36+ for that specific sample should not be used and is considered undefined. This particular case exists in the case of EDA and Clustering. It does not exist in the case of machine learning. The following observations can be made when looking at the Psat, Bo, and mole fractions of C1, C7+, C12+, C20+ and C36+: Since Bo is an Oil property, it is unlikely to have samples (with defined Bo) with Mole Fraction of C7+, C12+, C20+ or C36+ = 0; For samples with defined Bo, Mole Fraction of Methane is typically < 50% or 40%.

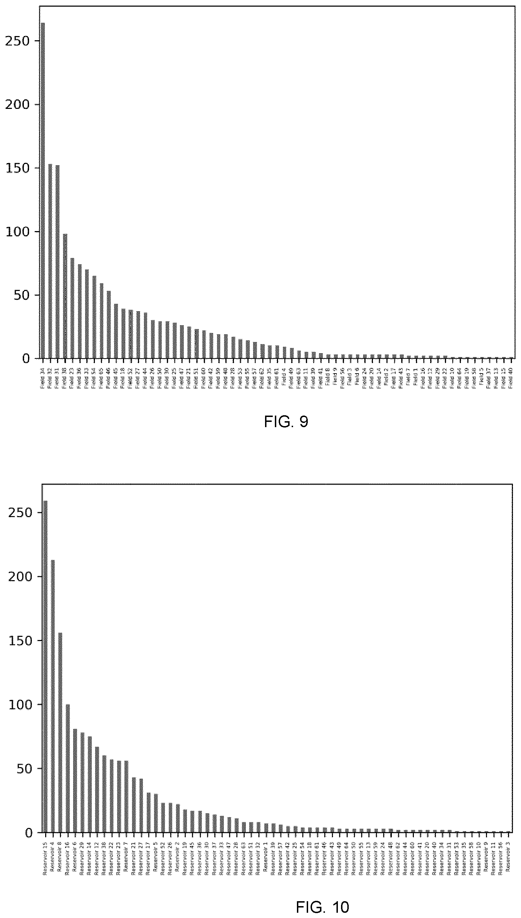

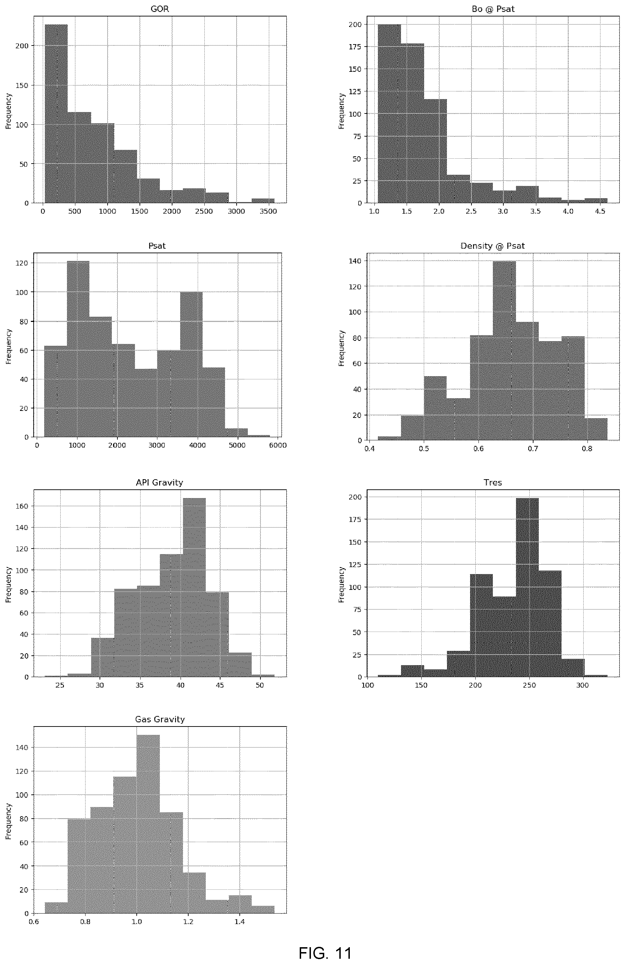

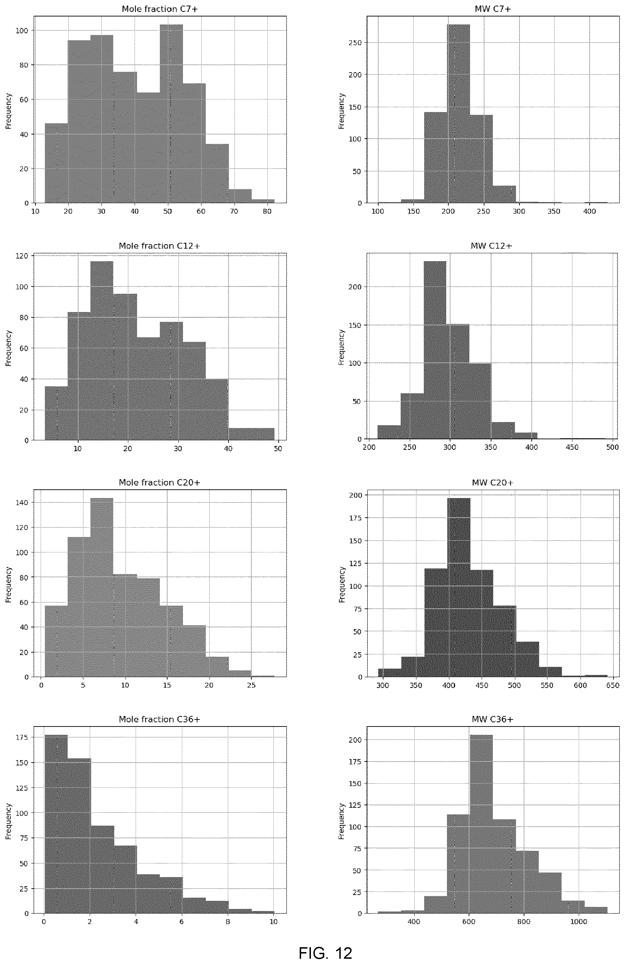

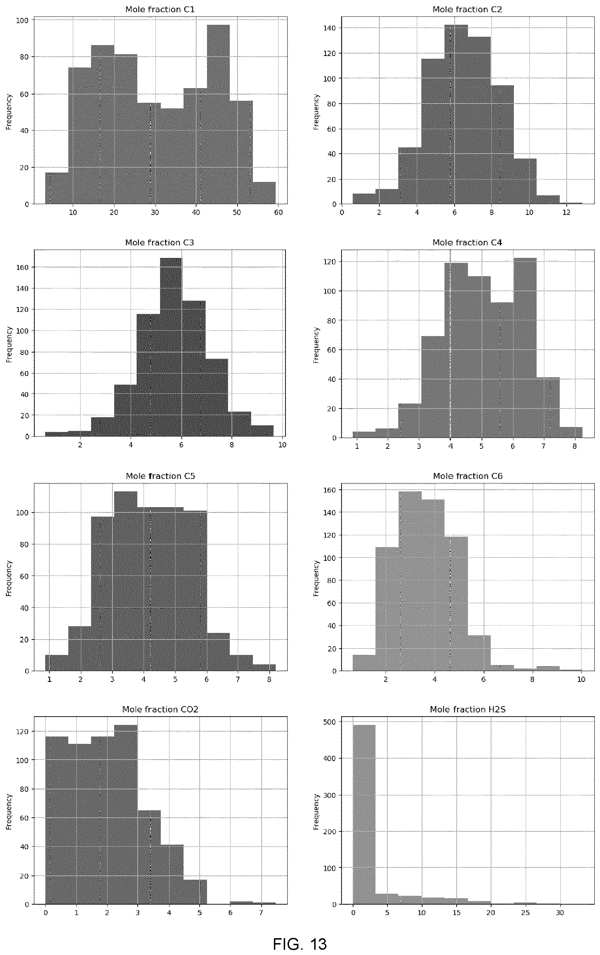

In the third step, data analysis is performed to generate statistics about the PVT data. This helps to better understand the distribution of the PVT data to be able to evaluate and interpret the PVT data. The following statistics and property distributions are automatically generated and reported using this framework: Distribution of the data across the fields; Distribution of the data across the reservoirs; Missing Values for each set of properties; Data distribution of Black Oil properties. E.g. Saturation Pressure, API Gravity, Reservoir Temperature, Saturated bubble point oil formation volume factor, GOR, Density at saturation pressure, Viscosity at saturation pressure, Data distribution of Compositional Properties (Mole fraction of C7+, Mole fraction of C12+, Mole fraction of C20+, Mole fraction of C36+, Molecular weight of C7+, Molecular weight of C12+, Molecular weight of C20+, Molecular weight of C36+). Examples of data statistics are presented in the Figs. 9, 10, 11, 12 and 13. Fig. 9 is a distribution over different fields of the data set used in the implementation and validation process, in particular the data belongs to a total of 65 fields. Fig. 10 is a distribution over different reservoirs of the data set used in the implementation and validation process, in particular the data belongs to a total of 64 reservoirs. Fig. 11 is a PVT Data set used in the implementation and validation process, in particular data statistics and black oil properties. Fig. 12 is a PVT Data set used in the implementation and validation process, particular data statistics, compositional properties, and heavy fractions. Fig. 13 is a PVT Data set used in the implementation and validation process, in particular data statistics, compositional properties, light and intermediate components.

With respect to the data prediction, algorithms are used to predict, using machine learning methods, any PVT property (Black oil or compositional) from any set of properties (black oil or compositional). Any of the properties in FLUID PROPERTIES can be predicted as a function of a sub-set of all other properties. The algorithm is generic and allows for adding any other property that can be obtained in a laboratory analysis (e.g. CCE, CVF, DL, MSS) at any pressure.

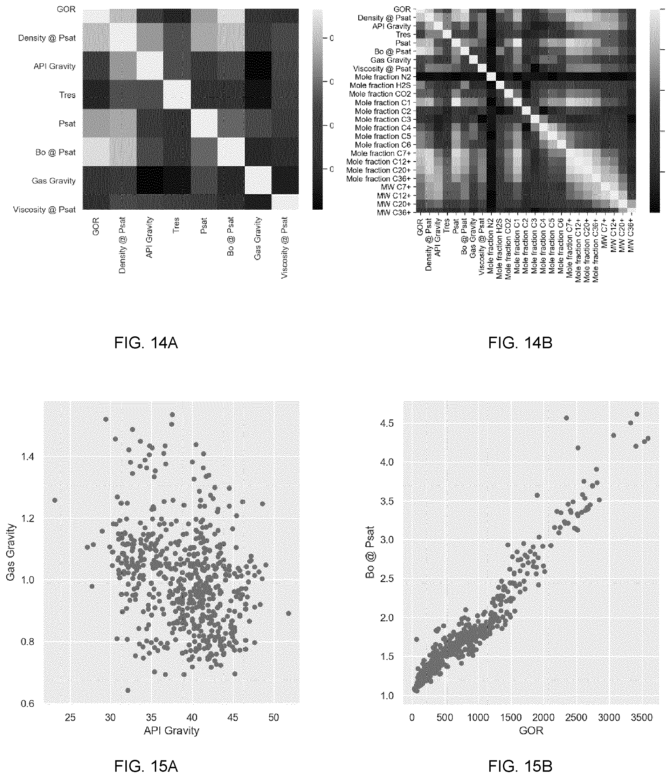

For validation, the present method 100 runs first machine learning to predict Psat and Bo@Psat from the same parameters used in the literature to predict these two parameters with correlations: temperature, gas oil ratio, stock tank API gravity and gas gravity. For understanding dependencies and eliminating irrelevant features, Exploratory Data Analysis (EDA) is used in the present invention to decrease the number of features used in PVT data prediction. EDA explores correlation between parameters to, systematically, eliminate parameters with no correlation from the machine learning models. Example results are presented in Figs. 14A, 14B, 15A, 15B, 16A, and 16B.

Figs. 14A and 14B are EDA Results applied to the PVT Data set used in the implementation and validation process, in particular Fig. 14A for black oil properties and Fig. 14B for black oil and compositional properties.

Figs. 15A and 15B are EDA Results, in particular black oil properties, in particular shows Fig. 15A the worst correlations and Fig. 15B the best correlations. The properties included in the EDA of Figs. 15A and 15B are illustrated in Fig. 14A.

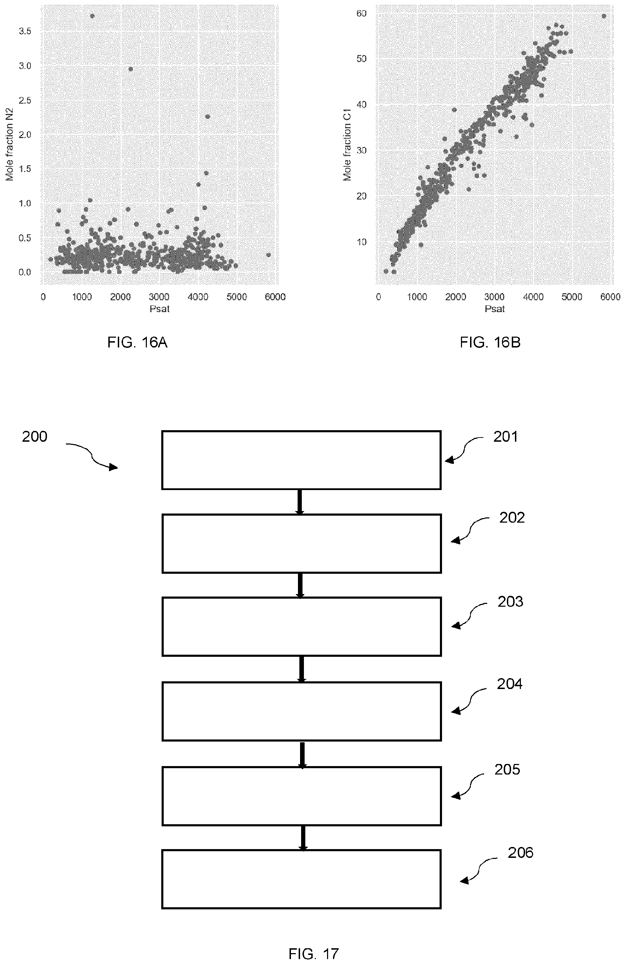

Figs. 16A and 16B are EDA Results, in particular compositional properties, in particular shows Fig. 16A the worst correlations and Fig. 16B the best correlations. The properties included in the EDA of Figs. 16A and 16B are illustrated in Fig. 14B.

With respect to improved accuracy prediction using the data clustering step 106, the data clustering step 106 is used to categorize the PVT data into families based on their collective behavior of their different features. The data clustering step 106 is performed for two main reasons: Using machine learning on different clusters instead of the whole PVT data set with the purpose of improving the predictive capability of different methods for different clusters; the EoS models available for different clusters will be compared with each other. In case similarity is found between these EoS models, one representative EoS model is selected per cluster. Once every sample belongs to a given cluster, the representative EoS model for that specific sample will be adopted for the specific sample.

The clustering 106 of method 100 can be performed using the following options: Clustering using black oil properties only; Clustering with compositional properties only; and Clustering with black oil and compositional properties. In all three cases, the clustering results can be used for machine learning to predict any of the black oil or compositional properties. In the following, the clustering to predict black oil properties is described to find out whether black oil properties can be predicted using compositional properties only without impacting the quality of the prediction.

For black oil clustering, the properties used for clustering are the following. Any other property could be used. Similarly, less properties or any other combination of properties can also be used.

Reservoir Temperature (Tres);
Solution gas oil ratio (GOR);
API Gravity;
Gas Gravity;
Saturation pressure (Psat);
Bo@Psat;
Viscosity @ Psat; and
Density @ Psat.

The number of samples used in this case is 593 samples. These are the samples with all above properties available.

For compositional properties based clustering, clustering is performed using completed compositional properties for which the compositional information is available for the full set of 1599 samples. This leads to a total of 1599 samples used in the clustering. Optimal number of clusters is this case is 5.

For black oil and compositional based clustering, clustering is performed using the following black oil properties:

Reservoir Temperature (Tres);
Solution gas oil ratio (GOR);
API Gravity;
Gas Gravity;
Saturation pressure (Psat);
Bo@Psat;
Viscosity @ Psat; and
Density @ Psat.

Completed compositional properties for which the compositional information is available for the 593 samples. The total number of samples that have all above data is 593.

Further, clusters can be associated with fields and reservoirs. The association between clusters of PVT data identified through the clustering step oil and gas fields / reservoirs with potentially different thermodynamic behavior.

Fig. 17 is a flow diagram of a computer-implemented method 200 for generating equations of state (EoS) for a plurality of hydrocarbon fluids in accordance to a third aspect of the present invention. The computer-implemented method 200 for generating equations of state (EoS) for a plurality of hydrocarbon fluids comprises the method step of delumping 201 pressure-volume-temperature (PVT) data for hydrocarbon fluid samples from a complete set of PVT data to one of a set of detailed fluid components, or to a common set of components and pseudo-components. The complete set of PVT data can be provided by the computer-implemented method 100 for predicting hydrocarbon fluid properties using machine-learning-based models in accordance to the first aspect of the present invention.

Further, the method 200 comprises the step of lumping 202 the PVT data from the complete set of PVT data into a pre-defined set of components and pre-defined set of pseudo-components to generate a plurality of equation of state (EoS) models. Further, the method 200 comprises generating 203 for the PVT samples on the PVT data set an EoS model using a same set of tuning parameters and thereby generating an EoS fluid model fingerprint for the hydrocarbon fluid samples. Further, the method 200 comprises the step of associating 204 properties of the hydrocarbon fluid samples with the generated EoS fluid model fingerprint.

The method 200, as shown in Fig. 17, comprises the step of training 205 machine learning models to predict an EoS fluid model fingerprint for new hydrocarbon fluid samples. The minimal set of PVT data for hydrocarbon fluid samples comprises black oil properties and compositional properties, wherein the black oil properties comprise at least one of reservoir temperature, solution gas-oil ratio, oil API gravity, gas gravity, dead oil viscosity, saturation pressure, saturated bubble point oil formation factor at saturation pressure, fluid density at reservoir conditions, fluid compressibility at reservoir conditions, viscosity at reservoir conditions, fluid density at reservoir conditions or any other black oil property, and wherein the compositional properties comprise at least one of mole fractions of the components, in particular N2, H2S, CO2, C1, C2, C3, C4, C5, C7, C8, and pseud-components, in particular C7+, C12+, C20+ and C36+ or any other pseudo-component as well as the molecular weight of the pseudo-components.

The step of associating 204 of method 200 further comprises the method step of clustering the selected items of the PVT data into a plurality of clusters for performing machine learning on each one of the plurality of clusters.

The method 200 further comprises the step of comparing 205 the results from clustering of the selected items of the PVT data with the plurality of equations of state (EoS) models and with heatmaps applying EoS models on the selected items of the PVT data.

The clustering 106, 204 of the selected items of the PVT data includes the step of identifying of clusters to which PVT data belong.

For delumping 201 into a common set of detailed components, as an input, the method step starts with SAMPLES that may or may not have the same set of DETAILED COMPOSTION or to a common set of components and pseudo-components. The chain-rule based machine learning of method 100 according to the first aspect of the present invention is used to complete all SAMPLES into the same set of detailed components or to a common set of components and pseudo-components.

As a first example, some samples would have the following set of components and pseudo-components: CO2, H2S, N2, C1, C2, C3, iC4, nC4, iC5, nC5, C6, C7+. Mole fraction of C7, C8, ..., C36+ and MW of C36+ will be predicted using ML models.

| | Detailed Set of Components | | | | | | | | | | | | | |
|---|---|---|---|---|---|---|---|---|---|---|---|---|---|---|
| | CO2 | H2S | N2 | C1 | C2 | C3 | iC4 | nC4 | iC5 | nC5 | C6 | C7 | ... | C36+ |
| Sample *1* | | | | | | | | | | | | | | |
| Sample *2* | | | | | | | | | | | | | | |
| ... | | | | | | | | | | | | | | |
| Sample *i* | | | | | | | | | | | | | | |
| ... | | | | | | | | | | | | | | |
| Sample *N_Samples* | | | | | | | | | | | | | | |

As a second example, some samples would have the following set of components and pseudo-components: CO2, H2S, N2, C1, C2, C3, iC4, nC4, iC5, nC5, C6, C7+. Mole fraction and MW of C12+, C20+ and C36+ will be predicted using ML models.

| | Predefined Set of Components and Pseudo-Components | | | | | | | | | | | | |
|---|---|---|---|---|---|---|---|---|---|---|---|---|---|
| | CO2 | H2S | N2 | C1 | C2 | C3 | C4 | C5 | C6 | C7+ | C12+ | C20+ | C36+ |
| Sample *1* | | | | | | | | | | | | | |
| Sample *2* | | | | | | | | | | | | | |
| ... | | | | | | | | | | | | | |
| Sample *i* | | | | | | | | | | | | | |

(continued)

| | Predefined Set of Components and Pseudo-Components | | | | | | | | | | | |
|---|---|---|---|---|---|---|---|---|---|---|---|---|
| | CO2 | H2S | N2 | C1 | C2 | C3 | C4 | C5 | C6 | C7+ | C12+ | C20+ | C36+ |
| ... | | | | | | | | | | | | |
| Sample *N_Samples* | | | | | | | | | | | | |

The step of lumping 202 into a predefined set of pseudo-components, in particular together with the step of delumping 201, are key to generate multiple EoSs structured in the same format to enable building machine learning models. The step of delumping 201 gives the flexibility, based on the delumping 201 of SAMPLES into the detailed set of components to lump 202 the samples components into any pre-defined set of components and pseudo-components. Multiple lumping schemes as provided with the present invention, may then be used and following steps may be repeated using each of the lumping schemes. Optionally, if the delumping 201 results on a pre-defined set of components and pseudo-components, the step of lumping 202 may be omitted.

With respect to the step of generating 203 for the PVT samples on the PVT data set an EoS model using the same set of tuning parameters and thereby generating an EoS fluid model fingerprint for the hydrocarbon fluid samples, for each validated sample in the data base 301, an EoS model is automatically calibrated using the same set of tuning parameters. This results in a set of values of these tuning parameters, for each PVT sample, which will be considered as the EoS fluid model fingerprint for each sample. The following steps are provided:

Decide (user) on an EoS to be used (e.g. 3-Parameter Peng-Robinson EoS).

Decide (user) on EXPERIMENTS and corresponding weights.

Decide (user) on TUNING PARAMETERS.

Decide (user) on the data-model matching tolerance.

For every sample, Si, in SAMPLES, tune an EoS within the prescribed tolerance.

For the results, a set of tuning parameters associated to the PVT sample Si, P1_Si, P2_Si, P3 _Si, ..., PN _Si, for every sample is provided.

| | TUNING PARAMETERS Resulting from Step 3 | | | | | | |
|---|---|---|---|---|---|---|---|
| | P1 | P2 | P3 | ... | Pj | ... | PN |
| Sample *1* | P1_1 | P2_1 | P3_1 | | Pj_1 | | PN_1 |
| Sample *2* | P1_2 | P2_2 | P3_2 | | Pj_2 | | PN_2 |
| ... | | | | | | | |
| Sample *i* | P1_i | P2_i | P3_i | | Pj_i | | PN_i |
| ... | | | | | | | |
| Sample *N_Samples* | P1_N_Samples | P2_N_Samples | P3_N_Samples | | Pj_N_Samples | | PN_N_Samples |

Further, with respect to the step of associating 204 properties of the hydrocarbon fluid samples with the generated EoS fluid model fingerprint, an association between samples properties and EoS fluid model fingerprint is build. This associating step 204 consists of training machine learning models to predict the EoS fluid model fingerprint for each sample and, therefore, EoS Models for any new PVT sample with the highest accuracy.

The use of machine learning to build models to predict P1, P2, P3, ...., PN based on the PVT samples properties (Compositional and Black oil) has the following input:

a. Complete set of compositional and Black oil for all PVT samples resulting from the method 100 according to the first aspect of the invention.

b. Results of the EoS tuning for all SAMPLES from Step 3: P1_Si, P2 _Si, P3 _Si, ..., PN _Si

The use of machine learning to build models to predict P1, P2, P3, ...., PN based on the PVT samples properties (Compositional and Black oil) has the following results: P1, P2, P3, ..., PN that, when used in an EoS provide a full match between the laboratory results and EoS results; Fully machine learned EoS models.

Further, the associating step 204 of associating properties of the hydrocarbon fluid samples with the generated EoS fluid model fingerprint can optionally include clustering. Accordingly, multiple machine learning models can be trained on the different clusters as described with the method 100 according to the first aspect of the invention. Several validation steps and insights will be drawn by comparing the trends from clustering of original samples, EoS models, and from heatmaps applying EoS models on all samples; all aiming towards improving the accuracy of the predicted EoS Model.

The method 200 according the third aspect of the invention can further include the step of validating. The step of validating can be applied to new samples or samples that have not been used in the machine learning step. The step of validation can be processed as following:

    1. A machine learned based EoS is build and loaded in a commercial PVT package

    2. Results are compared with laboratory data.

    3. Repeat Steps, as/if necessary, to optimize

        a. lumping scheme

        b. Set of tuning parameters.

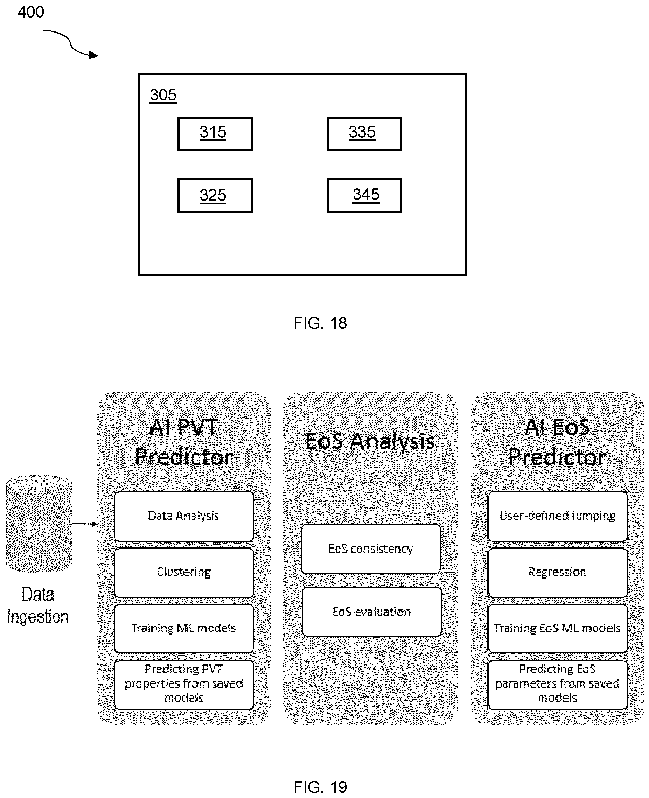

Fig. 18 is a schematic illustration of a system for for generating equations of state (EoS) for a plurality of hydrocarbon fluids in accordance to a fourth aspect of the present invention.

The system 400 for generating equations of state (EoS) for a plurality of hydrocarbon fluids comprises a first module 315, a second module 325, a third module 335 and a fourth module 345. The first module 315 delumps pressure-volume-temperature (PVT) data for hydrocarbon fluid samples from a complete set of PVT data to one of a set of detailed fluid components, or to a common set of components and pseudo-components. The second module 325 lumps the PVT data for hydrocarbon fluid sample into a pre-defined set of components and pre-defined set of pseudo-components to generate a plurality of equation of state (EoS) models. The third module 335 generates for the hydrocarbon fluid samples in a PVT data base an EoS model using a same set of tuning parameters and thereby generating an EoS fluid model fingerprint for the hydrocarbon fluid samples. The fourth module 345 associates properties of the hydrocarbon fluid samples with the generated EoS fluid model fingerprint.

Fig. 19 is an overview diagram of the hydrocarbon fluid properties and EoS prediction using machine learning subject of the present invention.

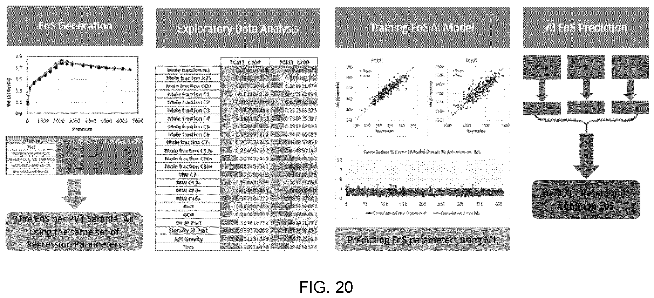

Fig. 20 is an overview of the machine-learning based EoS Workflow in accordance with the present invention. In the below an illustration use of the machine-learning based EoS is presented. A data set of 853 hydrocarbon liquid samples is used. These samples have a complete data set of laboratory experiments: saturation pressure, constant composition expansion, differential liberation and multi-stage separator. Part of these samples have a detailed composition up to C36+. The other part has composition to different cut-off below C36+ Therefore, machine learning is used to complete the composition of these samples in order to have the entire set of samples with the same detailed components.

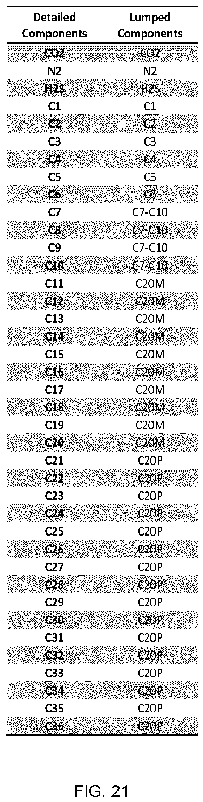

Fig. 21 illustrates the lumping scheme used in the process of creating the EoS model using regression. For the purpose of illustration, the detailed composition is lumped into 12 components and pseudo-components. As a result of this step, all 853 samples have the same set of lumped components and they are ready for regression. Different regression parameters where tested. As an illustration, here we present the results of regression using two parameters: PCRIT and TCRIT of C20P. The result of the regression on each of these samples is pair of PCRIT and TCRIT of C20P specific to that sample. The next step consists on using the results of regression in a machine learning model that predicts the EoS parameters; PCRIT and TCRIT of C20P in this specific case. Prior to doing so, Exploratory Data analysis (EDA) is used to select the set of parameters that have the highest impact on PCRIT and TCRIT of C20P.

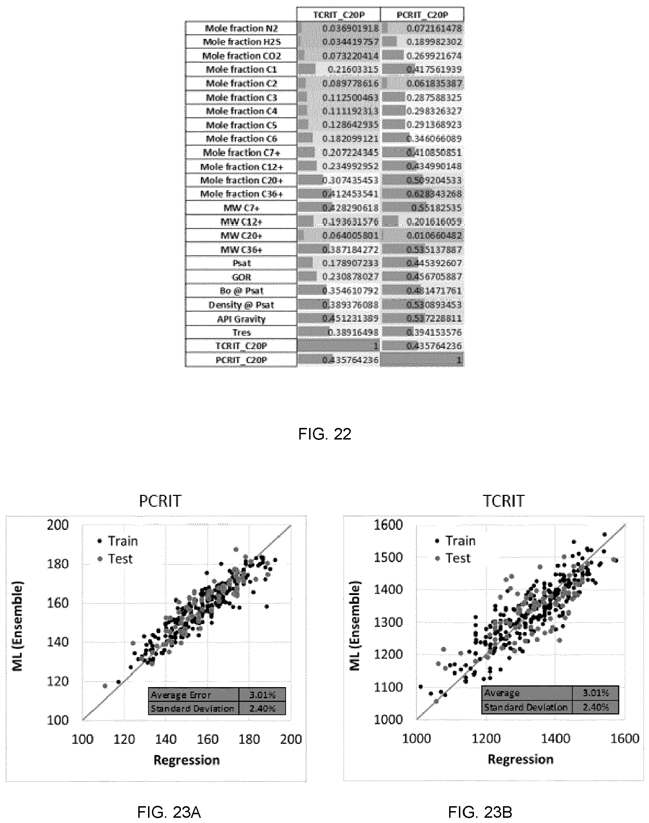

Fig. 22 is a heatmap resulting from Exploratory Data analysis (EDA) preceding machine learning to predict the EoS Parameters. TCRIT (C20P) and PCRIT (C20P) dependency on different BO and Compositional parameters. The results from EDA are the parameters to be used in machine learning. These are: Mole fraction of C1, C6, C7+, C12+, C20+, C36+, molecular weight of C7+, C12+, C36+, Psat, GOR, Bo@Psat, Density@Psat, API Gravity and Tres. Furthermore, chain rule is used: PCRIT of C20P is added to inputs to predict TCRIT of C20P.

Fig. 23A and 23B are an illustration of the predicted EoS parameters using machine learning when using Pcrit and Tcrit of C20P as regression parameters. The figure depicts Pcrit and Tcrit of C20P from machine learning vs. those obtained using conventional regression. The results of this step are machine learning predicted EoS parameters (PCRIT and TCRIT of C20P). These machine learning predicted parameters are then used in an EoS modeling tool (e.g. PVTi, PVTSim or any other tool) to predict the fluid properties and compare them with those obtained from laboratory measurement.

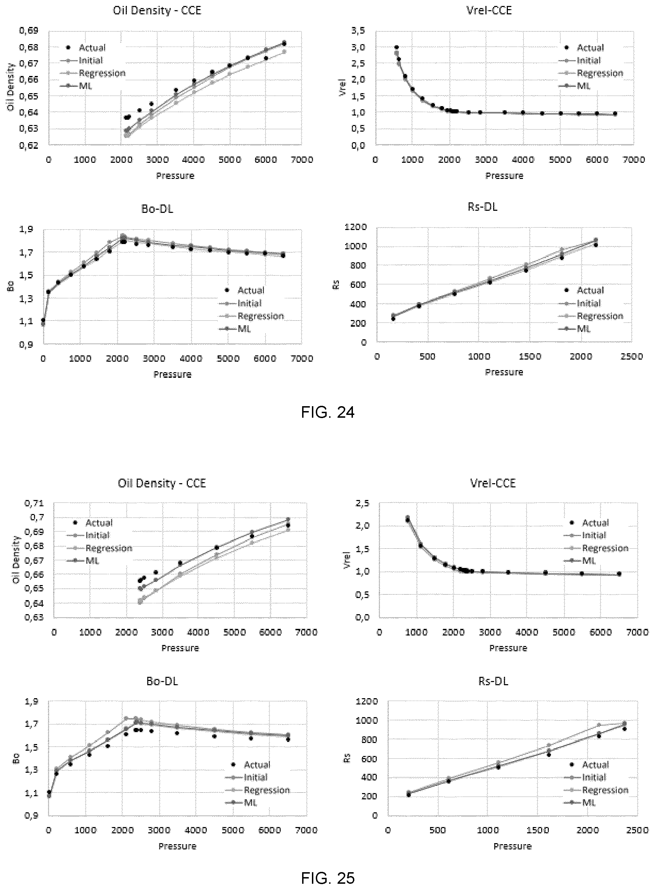

Fig. 24 illustrates example results of machine-learning based EoS - Sample 1, Field F1, Reservoir R1. The figure shows actual data, initial guess of EoS model without any regression, Conventional regression based EoS and ML based EoS.

Fig. 25 illustrates other example results of machine-learning based EoS - Sample 2, Field F2, Reservoir R2. The figure shows actual data, initial guess of EoS model without any regression, Conventional regression based EoS and ML based EoS.

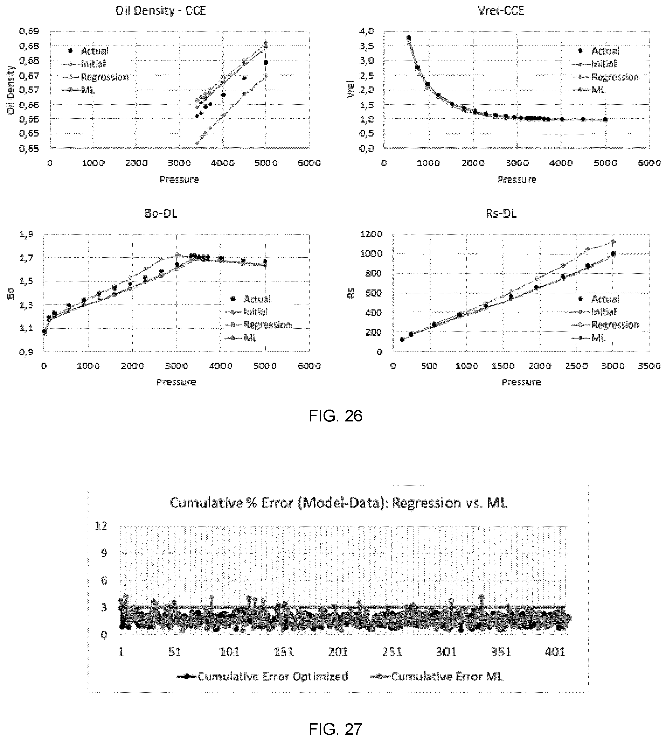

Fig. 26 illustrates other example results of machine-learning based EoS - Sample 3, Field F3, Reservoir R3. The figure shows actual data, initial guess of EoS model without any regression, Conventional regression based EoS and ML based EoS.

Fig. 27 illustrates machine-learning Based EoS - Error Analysis; Train + Test dataset. The EoS parameters resulting from ML are used in a PVT modeling tool to reproduce Laboratory experiments (CCE, DL and MSS). The figure shows the cumulative percentage error (EoS vs. Data) from conventional regression based EoS and machine learning based EoS.

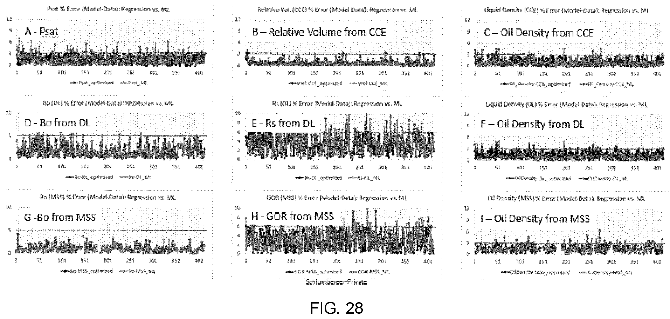

Fig. 28 illustrates machine-learning Based EoS - Error Analysis; Train + Test dataset. The EoS parameters resulting from ML are used in a PVT modeling tool to reproduce Laboratory experiments (CCE, DL and MSS). The figure shows the percentage error (EoS vs. Data) from conventional regression based EoS and machine learning based EoS for a selected set of fluid properties resulting from the above-mentioned experiments.

While only selected embodiments have been chosen to describe the present invention, it is apparent to the person skilled in the art from this disclosure that various changes and modifications can be made therein without deviating from the scope of the invention as defined in the attached claims.

**Claims**

1. A computer-implemented method (100) for predicting hydrocarbon fluid properties using machine-learning-based models, the method comprising:

   receiving (101) a minimal set of pressure-volume-temperature (PVT) data for hydrocarbon fluid samples from a PVT data base;
   reading (102) the minimal set of PVT data by a reader module;
   transforming (103) the minimal set of PVT data into a unified data structure by the reader module;
   selecting (104) items of the PVT data from the minimal set of PVT data by the reader module;
   processing (105) the selected items of the PVT data by a correlating module to identify a plurality of correlations in the selected items of the PVT data based on one or more of the fluid properties of the hydrocarbon fluid samples;
   clustering (106), using of at least one of a plurality of clustering algorithms, the selected items of the PVT data into a plurality of clusters by a clustering module; and
   performing (107) machine learning by a machine learning module on ones of the plurality of clusters to predict missing fluid properties in the minimal set of PVT data and thus to obtain a complete set of PVT data.

2. The computer-implemented method (100) according to claim 1, wherein the step of performing (107) machine learning by the machine learning module further comprises the step of predicting of fluid properties for future sets of PVT data for the hydrocarbon fluid samples.

3. The computer-implemented method (100) according to claim 1 or 2, further comprising the step of plotting (108) the machine learning predictions by the machine learning module.

4. The computer-implemented method (100) according to one of claims 1 to 3, further comprising the step of comparing (109) the identified plurality of correlation results with the machine learning predictions.

5. The method according to claim 1, wherein the clustering (106) further comprises the step of completing fluid composition including C12+, C20+ and C36+ mole fraction and molecular weight and/or completing black oil properties, including, in the following order: solution gas oil ratio (GOR), BO@Psat, and saturation pressure (Psat).

6. A computer-implemented method (200) for generating equations of state (EoS) for a plurality of hydrocarbon fluids, the method comprising:

delumping (201) pressure-volume-temperature (PVT) data for hydrocarbon fluid samples from a complete set of PVT data to one of a set of detailed fluid components, or to a common set of components and pseudo-components;

lumping (202) the PVT data from the complete set of PVT data into a pre-defined set of components and pre-defined set of pseudo-components to generate a plurality of equation of state (EoS) models;

generating (203) for the PVT samples on the PVT data set an EoS model using a same set of tuning parameters and thereby generating n EoS fluid model fingerprint for the hydrocarbon fluid samples; and

associating (204) properties of the hydrocarbon fluid samples with the generated EoS fluid model fingerprint.

7. The computer-implemented method (200) according to claim 6, further comprising training (205) machine learning models to predict an EoS fluid model fingerprint for new hydrocarbon fluid samples.

8. The computer-implemented method (100, 200) according to claim 1 or 6, wherein the minimal set of PVT data for hydrocarbon fluid samples comprises black oil properties and compositional properties, wherein the black oil properties comprise at least one of reservoir temperature, solution gas-oil ratio, oil API gravity, gas gravity, dead oil viscosity, saturation pressure, saturated bubble point oil formation factor at saturation pressure, fluid density at reservoir conditions, fluid compressibility at reservoir conditions, viscosity at reservoir conditions, fluid density at reservoir conditions or any other black oil property, and wherein the compositional properties comprise at least one of mole fractions of the components, in particular $N_2$, $H_2S$, $CO_2$, $C_1$, $C_2$, $C_3$, $C_4$, $C_5$, $C_7$, $C_8$, and pseud-components, in particular $C_{7+}$, $C_{12+}$, $C_{20+}$ and $C_{36+}$ or any other pseudo-component as well as the molecular weight of the pseudo-components.

9. The computer-implemented method (200) according to claim 6, wherein the step of associating (204) comprises the method step of clustering the selected items of the PVT data into a plurality of clusters for performing machine learning on each one of the plurality of clusters.

10. The computer-implemented method according to claim 9, further comprising the step of comparing (205) the results from clustering of the selected items of the PVT data with the plurality of equations of state (EoS) models and with heatmaps applying EoS models on the selected items of the PVT data.

11. The method according to any one of the claims 1 to 5 or claim 9, wherein the clustering (106, 204) of the selected items of the PVT data includes the step of identifying of clusters to which PVT data belong.

12. A system (300) for predicting hydrocarbon fluid properties using machine-learning-based models, the system comprising:

a pressure-volume-temperature (PVT) data base (301) providing a minimal set of pressure-volume-temperature (PVT) data for hydrocarbon fluid samples;

a reader module (302) for reading in a minimal set of PVT data, wherein the reader module is configured to transform the minimal set of PVT data into a unified data structure, and wherein the reader module is configured to select items of the PVT data from the minimal set of PVT data;

a correlating module (303) for processing the selected items of the PVT data to identify a plurality of correlations in the selected items of the PVT data based on one or more of the fluid properties of the hydrocarbon fluid samples;

a clustering module (304) for clustering, using of at least one of a plurality of clustering algorithms, the selected items of the PVT data into a plurality of clusters; and

a machine learning module (305) performing machine learning on ones of the plurality of clusters to predict missing fluid properties in the minimal set of PVT data and thus to obtain a complete set of PVT data.

13. A system (400) for generating equations of state (EoS) for a plurality of hydrocarbon fluids, the system comprising a first module (315) for delumping pressure-volume-temperature (PVT) data for hydrocarbon fluid samples from a complete set of PVT data to one of a set of detailed fluid components, or to a common set of components and pseudo-components,

a second module (325) for lumping the PVT data for hydrocarbon fluid sample into a pre-defined set of components and pre-defined set of pseudo-components to generate a plurality of equation of state (EoS) models,

a third module (335) for generating for the hydrocarbon fluid samples in a PVT data base an EoS model using a same set of tuning parameters and thereby generating an EoS fluid model fingerprint for the hydrocarbon fluid samples; and
a fourth module (345) configured to associate properties of the hydrocarbon fluid samples with the generated EoS fluid model fingerprint.

FIG. 1A

FIG. 1B

```
                        ┌──────────────┐
                        │ Correlations │
                        │              │
                        └──────────────┘

┌──────────┐   ┌──────────┐   ┌──────────┐   ┌──────────┐   ┌──────────┐
│ database │   │ Data     │   │Clustering│   │ Machine  │   │ Error    │
│          │   │ reader   │   │          │   │ Learning │   │ Analysis │
│          │   │ module   │   │          │   │ Models   │   │ and      │
│          │   │          │   │          │   │          │   │ Evaluation│
└──────────┘   └──────────┘   └──────────┘   └──────────┘   └──────────┘

                        ┌──────────────┐
                        │ Machine      │
                        │ Learning     │
                        │ Models       │
                        └──────────────┘

                        ┌──────────────┐
                        │ Reporting    │
                        │ and          │
                        │ Visualizing  │
                        └──────────────┘
```

FIG. 2

300

```
┌──────────┐      ┌─────────────────────────────────────┐
│          │      │  ┌────────┐                          │
│  301     │      │  │  302   │      ┌──────────────┐    │
│          │      │  └────────┘      │     305      │    │
│          │      │                  │              │    │
└──────────┘      │  ┌────────┐      │              │    │
                  │  │  303   │      │              │    │
                  │  └────────┘      └──────────────┘    │
                  │                                       │
                  │  ┌────────┐                          │
                  │  │  304   │                          │
                  │  └────────┘                          │
                  └─────────────────────────────────────┘
```

FIG. 3

```
                                                              ┌──────────────┐
                                                              │ Visualization│
                                                              │              │
                                                              └──────────────┘

┌──────────┐   ┌──────────┐  ┌──────────┐  ┌──────────┐  ┌──────────┐              ┌──────────────┐
│ database │──▶│ Receiving│─▶│ Structure│─▶│ Validate │─▶│ Outlier  │              │ Correlations │
│          │   │ Data     │  │ Data     │  │ /QC Data │  │ Detection│   ┌────────┐ └──────────────┘
│          │   │          │  │          │  │          │  │          │   │ PVT    │ ┌──────────────┐
└──────────┘   └──────────┘  └──────────┘  └──────────┘  └──────────┘   │ Data   │ │ Clustering   │
                                                                        │        │ └──────────────┘
                                                                        └────────┘ ┌──────────────┐
                                                                                   │ Machine      │
                                                                                   │ Learning     │
                                                                                   └──────────────┘
```

FIG. 4

```
                              ┌────────────┐ ┌────────────┐
                              │ Parameters │ │ CL         │         ┌────────┐  ┌────────┐
                              │            │ │ Algorithms │         │ PVT    │  │        │
                              └────────────┘ └────────────┘         │ Data   │  └────────┘
                                                                    │        │  ┌────────┐
                                                                    └────────┘  └────────┘
┌────────┐   ┌──────────┐ ┌────────┐ ┌──────────┐ ┌────────┐
│ PVT    │──▶│ Convert  │▶│normaliz│▶│ Dimensio │▶│ Cluster│        ┌────────────┐ ┌──────────────┐
│ Data   │   │ to CL    │ │ e      │ │ nality   │ │        │        │ Visualizati│ │ e.g. Graph   │
│        │   │ format   │ │        │ │ reduction│ │        │        │ on         │ └──────────────┘
└────────┘   └──────────┘ └────────┘ └──────────┘ └────────┘        └────────────┘
                                                                    ┌────────────┐ ┌──────────────┐
                                                                    │ Reportin   │ │ e.g. PDF     │
                                                                    │ g          │ │ file         │
                                                                    └────────────┘ └──────────────┘
                                                                                   ┌──────────────┐
                                                                                   │ e.g. Excel   │
                                                                                   │ file         │
                                                                                   └──────────────┘
```

FIG. 5

```
┌──────────────┐
│ Correlations │
│ from Library │
└──────┬───────┘
       │
       ▼
┌─────────┐   ┌──────────┐  ┌──────────┐  ┌────────┐
│  PVT    │   │ Select   │  │ Execute  │  │ Store  │
│  Data   │──▶│Correlations│▶│Correlations│▶│        │
└─────────┘   └──────────┘  └──────────┘  └────────┘
```

Error Analysis

Visualization → e.g. Graph

Reporting → e.g. PDF file
          → e.g. Excel file

FIG. 6

Input   output   EDA   Test percent   ML Algorithms   Clustering   Error measures

PVT Data → Convert to ML format → normalize → Feature Engineering → Train /test split → ML Algorithms → Evaluate best ML method

Visualization → e.g. Graph

Reporting → e.g. PDF file
          → e.g. Excel file

FIG. 7

| | Number |
|---|---|
| No missing data | 599 |
| ['gas_gravity'] | 316 |
| ['GOR', 'density_at_Psat', 'API_Gravity', 'Bo_Psat', 'viscosity_at_Psat'] | 206 |
| ['GOR', 'density_at_Psat', 'API_Gravity', 'Psat', 'Bo_Psat', 'viscosity_at_Psat'] | 92 |
| ['GOR', 'API_Gravity', 'Bo_Psat', 'viscosity_at_Psat'] | 87 |
| ['GOR', 'density_at_Psat', 'API_Gravity', 'Bo_Psat', 'gas_gravity', 'viscosity_at_Psat'] | 52 |
| ['Bo_Psat', 'viscosity_at_Psat'] | 47 |
| ['GOR', 'density_at_Psat', 'API_Gravity', 'Tres', 'Psat', 'Bo_Psat', 'viscosity_at_Psat'] | 26 |
| ['GOR', 'API_Gravity'] | 24 |
| ['Bo_Psat', 'gas_gravity'] | 22 |
| ['GOR', 'density_at_Psat', 'API_Gravity', 'Tres', 'Psat', 'Bo_Psat', 'gas_gravity', 'viscosity_at_Psat'] | 19 |
| ['GOR', 'density_at_Psat', 'API_Gravity', 'Psat', 'Bo_Psat', 'gas_gravity', 'viscosity_at_Psat'] | 17 |
| ['viscosity_at_Psat'] | 17 |
| ['Bo_Psat'] | 16 |
| ['GOR', 'API_Gravity', 'viscosity_at_Psat'] | 16 |
| ['Bo_Psat', 'gas_gravity', 'viscosity_at_Psat'] | 13 |
| ['GOR', 'API_Gravity', 'Bo_Psat'] | 12 |
| ['gas_gravity', 'viscosity_at_Psat'] | 12 |
| ['GOR', 'density_at_Psat', 'API_Gravity', 'Bo_Psat'] | 11 |
| ['gas_gravity', 'mole_fraction_C7_plus'] | 11 |
| ['GOR', 'API_Gravity', 'Bo_Psat', 'gas_gravity', 'viscosity_at_Psat'] | 8 |
| ['GOR', 'density_at_Psat', 'API_Gravity', 'Bo_Psat', 'gas_gravity', 'viscosity_at_Psat', 'mole_fraction_C7_plus'] | 8 |
| ['GOR', 'API_Gravity', 'Bo_Psat', 'gas_gravity'] | 7 |
| ['GOR', 'API_Gravity', 'gas_gravity', 'mole_fraction_C7_plus'] | 7 |
| ['GOR', 'density_at_Psat', 'API_Gravity', 'Bo_Psat', 'gas_gravity'] | 7 |
| ['Tres', 'gas_gravity'] | 7 |
| ['GOR', 'API_Gravity', 'gas_gravity'] | 6 |
| ['API_Gravity'] | 6 |
| ['density_at_Psat', 'Bo_Psat', 'viscosity_at_Psat'] | 6 |
| ['GOR', 'density_at_Psat', 'API_Gravity', 'Psat', 'Bo_Psat', 'gas_gravity', 'viscosity_at_Psat', 'mole_fraction_C7_plus'] | 4 |
| ['GOR', 'API_Gravity', 'gas_gravity', 'viscosity_at_Psat'] | 4 |
| ['GOR', 'API_Gravity', 'gas_gravity', 'viscosity_at_Psat', 'mole_fraction_C7_plus'] | 3 |
| ['Tres', 'Bo_Psat', 'viscosity_at_Psat'] | 3 |
| ['GOR', 'density_at_Psat', 'API_Gravity', 'Tres', 'Psat', 'Bo_Psat', 'gas_gravity', 'viscosity_at_Psat', 'mole_fraction_C7_plus'] | 2 |
| ['GOR', 'density_at_Psat', 'API_Gravity', 'Psat', 'Bo_Psat', 'viscosity_at_Psat', 'mole_fraction_C7_plus'] | 2 |
| ['Bo_Psat', 'gas_gravity', 'viscosity_at_Psat', 'mole_fraction_C7_plus'] | 2 |
| ['gas_gravity', 'viscosity_at_Psat', 'mole_fraction_C7_plus'] | 2 |
| ['GOR', 'API_Gravity', 'Bo_Psat', 'gas_gravity', 'viscosity_at_Psat', 'mole_fraction_C7_plus'] | 1 |
| ['GOR', 'density_at_Psat', 'API_Gravity', 'Tres', 'Bo_Psat', 'viscosity_at_Psat'] | 1 |
| ['API_Gravity', 'Bo_Psat'] | 1 |
| ['GOR', 'API_Gravity', 'Psat', 'Bo_Psat', 'viscosity_at_Psat'] | 1 |
| ['GOR', 'Bo_Psat', 'viscosity_at_Psat'] | 1 |
| ['GOR', 'density_at_Psat', 'API_Gravity', 'gas_gravity', 'mole_fraction_C7_plus'] | 1 |
| ['density_at_Psat', 'Bo_Psat'] | 1 |
| ['GOR', 'API_Gravity', 'Tres'] | 1 |
| ['density_at_Psat', 'gas_gravity'] | 1 |
| ['GOR', 'density_at_Psat', 'API_Gravity', 'Bo_Psat', 'gas_gravity', 'mole_fraction_C7_plus'] | 1 |
| ['density_at_Psat', 'Psat', 'Bo_Psat', 'gas_gravity', 'viscosity_at_Psat'] | 1 |
| ['density_at_Psat', 'Bo_Psat', 'gas_gravity', 'viscosity_at_Psat'] | 1 |

FIG. 8

FIG. 9

FIG. 10

FIG. 11

FIG. 12

FIG. 13

FIG. 14A

FIG. 14B

FIG. 15A

FIG. 15B

FIG. 16A

FIG. 16B

FIG. 17

400

305

315　　335

325　　345

FIG. 18

**AI PVT Predictor**

DB

Data Ingestion

Data Analysis

Clustering

Training ML models

Predicting PVT properties from saved models

**EoS Analysis**

EoS consistency

EoS evaluation

**AI EoS Predictor**

User-defined lumping

Regression

Training EoS ML models

Predicting EoS parameters from saved models

FIG. 19

FIG. 20

| Detailed Components | Lumped Components |
|---|---|
| CO2 | CO2 |
| N2 | N2 |
| H2S | H2S |
| C1 | C1 |
| C2 | C2 |
| C3 | C3 |
| C4 | C4 |
| C5 | C5 |
| C6 | C6 |
| C7 | C7-C10 |
| C8 | C7-C10 |
| C9 | C7-C10 |
| C10 | C7-C10 |
| C11 | C20M |
| C12 | C20M |
| C13 | C20M |
| C14 | C20M |
| C15 | C20M |
| C16 | C20M |
| C17 | C20M |
| C18 | C20M |
| C19 | C20M |
| C20 | C20M |
| C21 | C20P |
| C22 | C20P |
| C23 | C20P |
| C24 | C20P |
| C25 | C20P |
| C26 | C20P |
| C27 | C20P |
| C28 | C20P |
| C29 | C20P |
| C30 | C20P |
| C31 | C20P |
| C32 | C20P |
| C33 | C20P |
| C34 | C20P |
| C35 | C20P |
| C36 | C20P |

FIG. 21

| | TCRIT_C20P | PCRIT_C20P |
|---|---|---|
| Mole fraction N2 | 0.036901918 | 0.072161478 |
| Mole fraction H2S | 0.034419757 | 0.189982302 |
| Mole fraction CO2 | 0.073220414 | 0.269921674 |
| Mole fraction C1 | 0.21603315 | 0.417561939 |
| Mole fraction C2 | 0.089778616 | 0.061835387 |
| Mole fraction C3 | 0.112500463 | 0.287588325 |
| Mole fraction C4 | 0.111192313 | 0.298326327 |
| Mole fraction C5 | 0.128642935 | 0.291368923 |
| Mole fraction C6 | 0.182099121 | 0.346066089 |
| Mole fraction C7+ | 0.207224345 | 0.410850851 |
| Mole fraction C12+ | 0.234992952 | 0.434990148 |
| Mole fraction C20+ | 0.307435453 | 0.509204533 |
| Mole fraction C36+ | 0.412453541 | 0.628743268 |
| MW C7+ | 0.428290618 | 0.55182535 |
| MW C12+ | 0.193631576 | 0.201616059 |
| MW C20+ | 0.064005801 | 0.010660482 |
| MW C36+ | 0.387184272 | 0.535137887 |
| Psat | 0.178907233 | 0.445392607 |
| GOR | 0.230878027 | 0.456705887 |
| Bo @ Psat | 0.354610792 | 0.481471761 |
| Density @ Psat | 0.389376088 | 0.530893453 |
| API Gravity | 0.451231389 | 0.537228811 |
| Tres | 0.38916498 | 0.394153576 |
| TCRIT_C20P | 1 | 0.435764236 |
| PCRIT_C20P | 0.435764236 | 1 |

FIG. 22

FIG. 23A

FIG. 23B

FIG. 24

FIG. 25

FIG. 26

FIG. 27

FIG. 28

39

Europäisches
Patentamt

European
Patent Office

Office européen
des brevets

# EUROPEAN SEARCH REPORT

Application Number

EP 20 19 1806

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MESHAL ALMASHAN, NARUSUE YOSHIAKI, MORIKAWA HIROYUKI: "Estimating PVT Properties of Crude Oil Systems Based on a Boosted Decision Tree Regression Modelling Scheme with K-Means Clustering.", SPE/IATMI ASIA PACIFIC OIL & GAS CONFERENCE AND EXHIBITION, 25 October 2019 (2019-10-25), XP009525275, DOI: 10.2118/196453-MS * the whole document * | 1-5,8, 11,12 | INV. G16C20/30 G16C20/70 G16C60/00 G01N33/28 |
| X | OLOSO MUNIRUDEEN A ET AL: "Hybrid functional networks for oil reservoir PVT characterisation", EXPERT SYSTEMS WITH APPLICATIONS, OXFORD, GB, vol. 87, 12 June 2017 (2017-06-12), pages 363-369, XP085133613, ISSN: 0957-4174, DOI: 10.1016/J.ESWA.2017.06.014 * the whole document * | 1-5,8, 11,12 | |
| X | EL-SEBAKHY ET AL: "Data mining in forecasting PVT correlations of crude oil systems based on Type1 fuzzy logic inference systems", COMPUTERS & GEOSCIENCES, PERGAMON, AMSTERDAM, NL, vol. 35, no. 9, 1 September 2009 (2009-09-01), pages 1817-1826, XP026494999, ISSN: 0098-3004, DOI: 10.1016/J.CAGEO.2007.10.016 [retrieved on 2009-08-18] * the whole document * | 1-5,8, 11,12 | TECHNICAL FIELDS SEARCHED (IPC) G16C G01N |

-/--

~~The present search report has been drawn up for all claims~~

| Place of search | Date of completion of the search | Examiner |
|---|---|---|
| The Hague | 2 February 2021 | Denoual, Matthieu |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons

& : member of the same patent family, corresponding
     document

EPO FORM 1503 03.82 (P04C01)

Europäisches
Patentamt

European
Patent Office

Office européen
des brevets

## EUROPEAN SEARCH REPORT

Application Number

EP 20 19 1806

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | AMAR KHOUKHI ED - MARTÍN LAURA BLANCO: "Hybrid soft computing systems for reservoir PVT properties prediction", COMPUTERS & GEOSCIENCES, PERGAMON, AMSTERDAM, NL, vol. 44, 18 March 2012 (2012-03-18), pages 109-119, XP028519929, ISSN: 0098-3004, DOI: 10.1016/J.CAGEO.2012.03.016 [retrieved on 2012-04-01] * the whole document * ----- | 1-5,8, 11,12 | |

TECHNICAL FIELDS
SEARCHED      (IPC)

~~The present search report has been drawn up for all claims~~

| Place of search | Date of completion of the search | Examiner |
|---|---|---|
| The Hague | 2 February 2021 | Denoual, Matthieu |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt
European
Patent Office
Office européen
des brevets

Application Number

EP 20 19 1806

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-5, 12(completely); 8, 11(partially)

☐ The present supplementary European search report has been drawn up for those parts of the European patent application which relate to the invention first mentioned in the claims (Rule 164 (1) EPC).

**Europäisches Patentamt**
**European Patent Office**
**Office européen des brevets**

**LACK OF UNITY OF INVENTION**
**SHEET B**

Application Number

EP 20 19 1806

The Search Division considers that the present European patent application does not comply with the requirements of unity of invention and relates to several inventions or groups of inventions, namely:

```
1. claims: 1-5, 12(completely); 8, 11(partially)

      Method and system for predicting hydrocarbon fluids PVT
      data.
                        ---

2. claims: 6, 7, 9, 10, 13(completely); 8, 11(partially)

      Method and system of generating equations of state for
      hydrocarbon fluids.
                        ---
```

**REFERENCES CITED IN THE DESCRIPTION**

**Patent documents cited in the description**

- US 193519